# EUROPEAN PATENT APPLICATION

(11) **EP 4 752 551 A2**
(43) Date of publication of application: **03.06.2026**
(21) Application number: 26170086.8
(22) Date of filing: 11.06.2020
(51) Int. Cl.: G01N 33/68

(54) **ALLERGY ANTIGEN AND EPITOPE THEREOF**

(30) Priority: 13.06.2019 JP 2019110700
(62) Divisional of application: 20179456.7
(71) Applicant: Hoyu Co., Ltd., Nagoya-shi, Aichi 461-8650 (JP)
(72) Inventor: MATSUNAGA, Kayoko, Toyoake-shi, 470-1192 (JP); YAGAMI, Akiko, Toyoake-shi, 470-1192 (JP); AOKI, Yuji, Nagakute-shi, 480-1136 (JP); HASEGAWA, Erika, Nagakute-shi, 480-1136 (JP); SAKAI, Tomomi, Nagakute-shi, 480-1136 (JP)
(74) Representative: Mewburn Ellis LLP

(57) **Abstract**

The present invention provides novel antigens of an allergy to soybean, methods and kits for diagnosing an allergy to soybean, pharmaceutical compositions comprising such an antigen, soybeans or processed products of soybean in which such an antigen is eliminated, and a tester for determining the presence or absence of a soybean antigen in an object of interest. The present invention also relates to polypeptides comprising an epitope of an allergen, kits, compositions and methods for diagnosing an allergy, comprising such a polypeptide, pharmaceutical compositions comprising such a polypeptide, and food raw materials or edible processed products in which an antigen comprising such a polypeptide is eliminated or reduced, or the polypeptide is cleaved or removed. The present invention further relates to a tester for determining the presence or absence of an antigen in an object of interest.

## Description

The present invention relates to a novel antigen of an allergy to wheat, etc. The present invention also relates to a kit, a composition, and a method for diagnosing allergy to wheat, etc. The present invention also relates to a composition comprising such an antigen and raw materials or processed products in which such an antigen is eliminated or reduced. The present invention further relates to a tester composition for determining the presence or absence of a wheat antigen in an object of interest.

The present invention also relates to an antigen of a polypeptide comprising an epitope. The present invention also relates to a kit, a composition and a method for diagnosing an allergy, comprising such a polypeptide. The present invention also relates to a method for providing an indicator for diagnosing an allergy in a subject. The present invention also relates to a composition comprising such a polypeptide, and a raw material or a processed product in which such a polypeptide is eliminated or reduced. The present invention further relates to a method for producing a raw material and a processed product in which such a polypeptide is eliminated or reduced. The present invention further relates to a tester composition for determining the presence or absence of an antigen comprising such a polypeptide in an object of interest.

In serum and tissues of allergic patients, IgE antibodies specific to particular antigens (hereinafter also referred to as allergens) are produced. Physiological consequences caused by interaction between such IgE antibodies and such particular antigens elicit allergic reactions. The antigens refer to food or cooking ingredients, etc. that cause allergic symptoms in a broad sense, and refer to proteins (hereinafter also referred to as allergen components) contained in food or cooking ingredients, etc. to which specific IgE antibodies bind in a narrow sense.

In the process of production of conventional allergy testing agents, antigen reagents are commonly prepared simply by grinding a candidate allergenic food, cooking ingredient or the like (Patent Literature 1). For this reason, the only case where conventional allergy tests have permitted detection of a positive allergic reaction is when in a conventional antigen reagent containing many types of allergen components, an allergen component is present in an amount exceeding a threshold that allows determination of a positive reaction for binding to an IgE antibody, and diagnosis efficiency was not sufficiently high.

Some allergen components have been suggested for allergen candidate food or cooking ingredients, and have also been commercialized as testing kits. While it is necessary to exhaustively identify allergen components in order to enhance the reliability of allergy tests, the patient detection rate by the measurement of such allergenic components is far from sufficient. Identification of novel allergens in wheat is very important not only for increasing the precision of diagnosis, but also for determining targets of low allergenic food, low allergenic cooking ingredients and therapeutic agents.

Meanwhile, in the field of protein separation and purification, a method for separating and purifying many different proteins from a small amount of sample has been used in recent years, which is more specifically a two-dimensional electrophoresis consisting of isoelectric focusing in the first dimension, followed by SDS-PAGE (sodium dodecyl sulfate-polyacrylamide gel electrophoresis) in the second dimension. The present applicant has conventionally developed some 2D electrophoresis methods with high separation ability (Patent Literature 2-5).

Allergen-specific IgE antibodies recognize and bind to epitopes that are particular amino acid sequences in allergen components. However, only a slight number of analyses have been made on epitopes as to the allergen components (Non Patent Literature 1), but such analyses are still totally quite rare. Furthermore, any kit for diagnosing an allergy using a polypeptide comprising an epitope has not yet emerged in the market.
PTL1: Japanese Patent Application Publication No. JP 2002-286716
PTL2: Japanese Patent Application Publication No. JP 2011-33544
PTL3: Japanese Patent Application Publication No. JP 2011-33546
PTL4: Japanese Patent Application Publication No. JP 2011-33547
PTL5: Japanese Patent Application Publication No. JP 2011-33548
NPL 1: Matsuo, H., et al., J. Biol. Chem., (2004), Vol.279, No.13, pp.12135-12140

The present invention provides novel antigens of an allergy which are proteins. The present invention also provides methods and kits for diagnosing allergy, comprising such an antigen. The present invention also provides compositions comprising such an antigen and raw materials or processed products in which such an antigen is eliminated or reduced. The present invention further provides tester compositions for determining the presence or absence of an antigen in an object of interest.

The present invention also provides antigens of polypeptides comprising an epitope. The present invention also provides kits, compositions and methods for diagnosing an allergy, comprising such a polypeptide. The present invention also provides methods for providing an indicator for diagnosing an allergy in a subject. The present invention also provides compositions comprising such a polypeptide, and raw materials or processed products in which an antigen comprising such a polypeptide is eliminated or reduced. The present invention further relates to methods for producing a raw material and a processed product in which such an antigen is eliminated or reduced. The present invention further relates to tester compositions for determining the presence or absence of an antigen comprising such a polypeptide in an object of interest.

In order to solve the aforementioned problems, the present inventors had made intensive studies to identify causative antigens of an allergy to wheat. As a result, the inventors succeeded in identifying novel antigens of proteins to which an IgE antibody in the serum of a patient who is allergic to wheat specifically binds.

Since the epitopes have a relatively short amino acid sequence, the IgE antibodies are capable of binding to different allergen components if the same amino acid sequence is present in the different allergen components. Because different allergen components have a common epitope so that IgE antibodies from allergic patients bind to both of them, the antigens have cross-reactivity. Thus, the epitopes defined in the present invention enable diagnosis or treatment of an allergy including cross-reactivity, and detection of a plurality of allergen components comprising the epitopes, etc.

As referred to herein, the "antigen" is used in meanings including both an antigen in a narrow sense which is a protein and an "epitope" derived from the protein, unless otherwise specified. The "antigen" is used in any meaning of the protein or the epitope derived from the protein as specified.

The present invention has been completed based on the aforementioned finding. The present invention includes the following embodiments, but the present invention is not limited to them.
[Embodiment 1] A kit for diagnosing an allergy, comprising at least one of the following polypeptides (E4), (E9), (E11), (E19), (E31), (E36), (E37), (E43) and (E44):
   (E4) a polypeptide comprising at least one amino acid sequence selected from the amino acid sequences of SEQ ID NOs: 281-341 and 343-345;
   (E9) a polypeptide comprising at least one amino acid sequence selected from the amino acid sequences of SEQ ID NOs: 640-705 and 2885-2888;
   (E11) a polypeptide comprising at least one amino acid sequence selected from the amino acid sequences of SEQ ID NOs: 776-898 and 2892;
   (E19) a polypeptide comprising at least one amino acid sequence selected from the amino acid sequences of SEQ ID NOs: 1257-1296;
   (E31) a polypeptide comprising at least one amino acid sequence selected from the amino acid sequences of SEQ ID NOs: 1895-1976, 2912 and 2913;
   (E36) a polypeptide comprising at least one amino acid sequence selected from the amino acid sequences of SEQ ID NOs: 2154-2216;
   (E37) a polypeptide comprising at least one amino acid sequence selected from the amino acid sequences of SEQ ID NOs: 2217-2264 and 2917-2921;
   (E43) a polypeptide comprising at least one amino acid sequence selected from the amino acid sequences of SEQ ID NOs: 2545-2612; and
   (E44) a polypeptide comprising at least one amino acid sequence selected from the amino acid sequences of SEQ ID NOs: 2613-2630.
[Embodiment 2] A composition for diagnosing an allergy, the composition comprising at least one of polypeptides (E4), (E9), (E11), (E19), (E31), (E36), (E37), (E43) and (E44) according to Embodiment 1.
[Embodiment 3] A method for providing an indicator for diagnosing an allergy in a subject, the method comprising the steps of:
   (i) contacting a sample obtained from the subject with an antigen, wherein the sample is a solution comprising an IgE antibody;
   (ii) detecting binding between the IgE antibody present in the sample obtained from the subject and the antigen; and
   (iii) when the binding between the IgE antibody in the subject and the antigen is detected, an indicator of the fact that the subject is allergic is provided;
   wherein the antigen is at least one of polypeptides (E4), (E9), (E11), (E19), (E31), (E36), (E37), (E43) and (E44) according to Embodiment 1.
[Embodiment 4] An antigen which is at least one of polypeptides (E4), (E9), (E11), (E19), (E31), (E36), (E37), (E43) and (E44) according to Embodiment 1 and is causative of an allergy.
[Embodiment 5] A composition comprising at least one antigen according to Embodiment 4.
[Embodiment 6] The composition according to Embodiment 5, wherein the composition is intended for the treatment of an allergy.
[Embodiment 7] A tester composition for determining the presence or absence of an antigen in a subject, the tester composition comprising an antibody that binds to at least one of polypeptides (E4), (E9), (E11), (E19), (E31), (E36), (E37), (E43) and (E44) according to Embodiment 1.
[Embodiment 8] A tester composition for determining the presence or absence of an antigen in an object of interest, the tester composition comprising at least one primer comprising a portion of the nucleotide sequence of a nucleic acid encoding any of polypeptides (E4), (E9), (E11), (E19), (E31), (E36), (E37), (E43) and (E44) according to Embodiment 1, and/or a portion of a complementary strand thereof.
[Embodiment 9] A tester composition for determining the presence or absence of an IgE antibody in a subject, the tester composition comprising polypeptides (E4), (E9), (E11), (E19), (E31), (E36), (E37), (E43) and (E44) according to Embodiment 1.
[Embodiment 10] A method for determining the presence or absence of polypeptides (E4), (E9), (E11), (E19), (E31), (E36), (E37), (E43) and (E44) according to Embodiment 1 in a raw material or a processed product, comprising detecting the polypeptides (E4), (E9), (E11), (E19), (E31), (E36), (E37), (E43) and (E44) according to Embodiment 1 in the raw material or the processed product.
[Embodiment 11] A raw material or a processed product in which an antigen is eliminated or reduced, wherein the antigen is at least one of polypeptides (E4), (E9), (E11), (E19), (E31), (E36), (E37), (E43) and (E44) according to Embodiment 1.
[Embodiment 12] A method for producing a raw material or a processed product in which an antigen is eliminated or reduced, the method comprising the step of confirming that the antigen is eliminated or reduced, in a production process of the processed product, wherein the antigen is at least one of polypeptides (E4), (E9), (E11), (E19), (E31), (E36), (E37), (E43) and (E44) according to Embodiment 1.

The present invention can provide novel antigens of an allergy (e.g., an allergy to wheat). Since the novel allergen components that trigger an allergy were identified according to this invention, this invention can provide highly sensitive methods and kits for diagnosing an allergy, compositions comprising such an antigen, raw materials or processed products in which such an antigen is eliminated or reduced, and tester compositions for determining the presence or absence of an antigen in an object of interest.

The present invention can provide antigens of novel polypeptides comprising an epitope of a protein antigen. Use of the polypeptide of the present invention enables provision of highly sensitive kits, compositions and methods for diagnosing an allergy (e.g., an allergy to wheat), compositions comprising such a polypeptide, tester compositions for determining the presence or absence of an antigen comprising such a polypeptide in an object of interest, and raw materials or processed products in which such a polypeptide is eliminated or reduced, and a method for producing the raw materials and the processed products.
Fig. 1 is a photograph of a gel showing a protein electrophoretic pattern in two-dimensional electrophoresis of proteins contained in wheat. The bands at the left of the photograph are bands of molecular weight markers, and the numeric values at the left of the photograph are respective molecular weights (KDa) of the molecular weight markers. The numeric values at the top of the photograph represent isoelectric points.
Fig. 2 is a photograph of an immunoblot of a two-dimensional electrophoretic pattern of proteins contained in wheat stained with serum of a wheat-allergic patient. Spots 1 to 16 where an IgE antibody in the serum of the wheat-allergic patient specifically reacted as compared with healthy subjects are each enclosed in a white line. WDEIA cases in wheat-allergic patients are represented by P2 to P36, children of cases with general immediate type allergy to wheat, which requires no exercise for its development, are represented by P50 to P57, and adults are represented by G1 to G9.
Fig. 3 shows results of examining cross-reactivity of peptides having the amino acid sequence of each epitope by ELISA using serum of a patient associated with an allegy to wheat.
Fig. 4 shows results of examining cross-reactivity of peptides having the amino acid sequence of each epitope by ELISA using serum of a patient associated with an allegy to wheat.
Fig. 5 shows results of examining cross-reactivity of peptides having the amino acid sequence of each epitope by ELISA using serum of a patient associated with an allegy to wheat.
Fig. 6 shows results of examining cross-reactivity of peptides having the amino acid sequence of each epitope by ELISA using serum of a patient associated with an allegy to wheat.

The present invention will be described in detail below, but the present invention is not limited to them.

Unless otherwise defined herein, all scientific and technical terms used in relation to the present invention shall have meanings commonly understood by those skilled in the art.

As referred to herein, the "allergy" refers to the state in which, when a certain antigen enters the body of a living individual sensitized to said antigen, the living individual shows a hypersensitive reaction detrimental to him/her. An allergic reaction can be produced upon contact with an antigen or consumption of the antigen. Here, the contact refers to touch to an object and, particularly, as for the human body, refers to attachment to the skin, the mucosa (eyes, lips, etc.) or the like. The consumption refers to incorporation into the body and refers to incorporation by inhalation or through an oral route, etc. In general, allergic reactions caused by consumption of foods are particularly referred to as food allergies. In a preferred embodiment, the allergy may be a food allergy. In blood and tissues of individuals with many food-allergic diseases, IgE antibodies specific to antigens are produced. IgE antibodies bind to mast cells or basophils. When an antigen specific to such an IgE antibody enters again the body of a patient with an allergic disease, said antigen combines with the IgE antibody bound to mast cells or basophils, resulting in physiological effects of IgE antibody-antigen interaction. Examples of such physiological effects include release of histamine, serotonin, heparin, eosinophil chemotactic factors, leucotrienes, or the like. These released substances provoke an allergic reaction resulting from the combination of an IgE antibody with particular antigens. Specifically, IgE antibodies recognize and bind to epitopes that are particular amino acid sequences in particular antigens. Allergic reactions caused by such antigens occur through the aforementioned pathway.

In the present invention, the allergy of interest is not particularly limited as long as it is an allergy to an allergen (antigen) comprising an epitope to be used. In one embodiment, the allergen includes grain, seafood, fruits, vegetables, nuts (seeds), edible grass, meat, milk, dairy products and the like that are consumed by living individuals (particularly, humans), or parasites and the like that parasitize living individuals (particularly, humans).

The grain is a generic name for cooking ingredients obtained from plants, and means edible seeds composed mainly of starch. The grain refers to only seeds of crops of the family *Poaceae* (cereal crops) in a narrow sense and includes these seeds as well as seeds of crops of the family *Leguminosae* (bean crops) or seeds of crops of other families in a broad sense. As for the grain in the broad sense, dicotyledonous seeds available as grain are collectively called pseudocereals because of their similarity to seeds of cereal crops (seeds of crops of the family *Poaceae* which are monocotyledonous plants). The pseudocereals include buckwheat (family *Polygonaceae*), amaranth (family *Amaranthaceae*), quinua (quinoa; family *Chenopodiaceae*) and the like.

In the present invention, the grain of the family *Poaceae* is not limited and includes bread wheat (*Triticum aestivum*), barley, rye, oat, timothy, orchard grass, vernal grass, common millet, foxtail millet, Japanese millet, corn, Job's tears and the like. The grain of the family *Polygonaceae* includes, for example, buckwheat (*Fagopyrum esculentum*)*.* In one embodiment, an antigen (allergen) is derived from grain. In one embodiment, an allergen is derived from grain of the family *Poaceae.* In one embodiment, an allergen is derived from wheat.

The seafood is not limited and includes shrimps, crabs and the like belonging to the order *Decapoda.* Most of organisms generally recognized as "crustaceans" are included in the order *Decapoda.* The seafood is not limited and also includes squids and octopuses belonging to the order *Teuthida* or the order *Octopoda.* The seafood further includes fishes belonging to the family *Scombridae* or the family *Gadidae.* Furthermore, the seafood also includes clams of the family *Veneridae.*

The fruits are not limited and include, for example, fruits belonging to the family *Actinidiaceae,* the family *Bromeliaceae,* the family *Anacardiaceae,* the family *Cucurbitaceae,* the family *Musaceae,* the family *Rutaceae,* and the family *Rosaceae.* The vegetables include, for example, fruits belonging to the family *Solanaceae,* the family *Cucurbitaceae,* and the family *Lauraceae.* The nuts (seeds) include, for example, nuts (seeds) belonging to the family *Anacardiaceae,* the family *Rosaceae,* and the family *Juglandaceae.* The edible grass includes, for example, edible grass belonging to the family *Compositae.* The grain includes, for example, grain belonging to the family Poaceae and the family *Polygonaceae.*

The type of the meat is not particularly limited. In one embodiment, meat of birds (chicken meat, canard viande, etc.), pork, beef, sheep meat, and the like are included therein. In one embodiment, meat of a bird is used. In one embodiment, meat of chicken (*Gallus gallus*) is used.

The origin of the milk is not particularly limited. In one embodiment, milk derived from a cow, a goat, sheep or the like is included therein. In one embodiment, milk of a cow (*Bos taurus*) is used. The dairy products are processed products of milk. The dairy products are not limited and include butter, fresh cream, cheese, yogurt, and ice cream.

The parasites are not limited and include, for example, parasites of the family *Anisakidae.* The parasites of the family *Anisakidae* include anisakis (*Anisakis simplex*)*.*

As referred to herein, the allergy refers to the state in which an individual has an allergic reaction caused by proteins, etc. present in raw materials or processed products (e.g., wheat or processed products of wheat)) which act as an antigen. The allergy can produce an allergic reaction upon contact with an antigen contained in raw materials or processed products (e.g., wheat or processed products of wheat) or consumption of the antigen. In general, allergic reactions caused by consumption of foods are particularly referred to as food allergies. The allergy to wheat may be a food allergy.

As referred to herein, the "antigen" refers to a substance that provokes an allergic reaction. A protein contained in raw materials such as cooking ingredients is also referred to as an allergen component. The antigen is preferably a protein.

As referred to herein, the protein is a molecule having a structure in which naturally occurring amino acids are joined together by peptide bond. The number of amino acids present in a protein is not particularly limited. As referred to herein, the term "polypeptide" also means a molecule having a structure in which naturally occurring amino acids are joined together by peptide bond. The number of amino acids present in a polypeptide is not particularly limited. The "polypeptide" conceptually includes the "protein". Also, polypeptides having about 2 to 50 amino acids joined together by peptide bond are in some cases called "peptides", especially.

In the case where amino acids can form different enantiomers, the amino acids are understood to form an L-enantiomer, unless otherwise indicated. The amino acid sequences of proteins, polypeptides, or peptides as used herein are represented by one-letter symbols of amino acids in accordance with standard usage and the notational convention commonly used in the art. The leftward direction represents the amino-terminal direction, and the rightward direction represents the carboxy-terminal direction. In the one-letter symbols of amino acids, X can be any substance having an amino group and a carboxyl group that can bind to amino acids at both ends, and particularly represents that any of 20 types of naturally occurring amino acids are acceptable.

An alanine scanning technique (or an "alanine/glycine scanning" technique) is a method in which variants in which residues in a protein are varied one by one to alanine (or glycine when the original amino acid is alanine) are prepared to identify site-specific residues important for the structure or function of the protein. Residues at positions where binding activity against IgE antibodies from patients remain even after the variation to alanine (or glycine when the original amino acid is alanine) are not important for the binding activity against IgE antibodies, and the binding activity remains even after exchange of these residues with other amino acids. The binding activity against IgE antibodies refers to detected binding and reaction between an epitope of interest and an IgE antibody. The residue of X in the Sequence Listing of the present application is an amino acid residue at a site where binding activity against IgE antibodies from allergic patients remains even after substitution by alanine (or glycine when the original amino acid is alanine) in alanine/glycine scanning described in Example 4. It is well known to those skilled in the art that even when such a site is substituted by any other amino acids, it is highly probable that this binding activity against IgE antibodies remains. Specifically, such a residue can be substituted by not only alanine or glycine but also any given amino acid residue other than alanine.

The binding and maintenance between IgE and an antigen (an epitope) are important for a subsequent allergic reaction, and this binding and maintenance are brought about by the electric charge, hydrophobic bond, hydrogen bond, and aromatic interaction of the epitope. Binding and maintenance that can be attained even if these are lost by change to alanine or glycine mean that the amino acid is not important.

### Identification of antigens

Proteins contained in wheat (bread wheat (*Triticum aestivum*)) were subjected to two-dimensional electrophoresis under the conditions described below to identify an antigen of an allergy to wheat.

The electrophoresis in the first dimension was isoelectric focusing, which was performed using isoelectric focusing gels with a gel-strip length of 5 to 10 cm and a gel pH range of 3 to 10. The pH gradient of the gels in the direction of electrophoresis was as follows: when the total gel-strip length is taken as 1, the gel-strip length up to pH 5 is taken as "a", the gel-strip length from pH 5 to 7 is taken as "b", and the gel-strip length above pH 7 is taken as "c", "a" is in the range of 0.15 to 0.3, "b" is in the range of 0.4 to 0.7, and "c" is in the range of 0.15 to 0.3. More specifically, the isoelectric focusing was performed using the IPG gels, Immobiline Drystrip (pH3-10NL), produced by GE Healthcare Bio-Sciences Corporation (hereinafter abbreviated as "GE"). The electrophoresis system used was IPGphor produced by GE. The maximum current of the electrophoresis system was limited to 75 µA per gel strip. The voltage program adopted to perform the first-dimensional isoelectric focusing was as follows: (1) a constant voltage step was performed at a constant voltage of 300 V until the volt-hours reached 750 Vhr (the current variation width during electrophoresis for 30 minutes before the end of this step was 5 µA); (2) the voltage was increased gradually to 1000 V for 300 Vhr; (3) the voltage was further increased gradually to 5000 V for 4500 Vhr; and then (4) the voltage was held at a constant voltage of 5000 V until the total Vhr reached 12000.

The electrophoresis in the second dimension was SDS-PAGE, which was performed using polyacrylamide gels whose gel concentration at the distal end in the direction of electrophoresis was set to 3 to 6% and whose gel concentration at the proximal end was set to a higher value than that at the distal end. More specifically, the SDS-PAGE was performed using NuPAGE 4-12% Bis-Tris Gels (IPG well, Mini, 1 mm) produced by Life Technologies. The electrophoresis system used was XCell SureLock Mini-Cell produced by Life Technologies. The electrophoresis was run at a constant voltage of 200 V for about 45 minutes using an electrophoresis buffer composed of 50 mM MOPS, 50 mM Tris base, 0.1% (w/v) SDS and ImM EDTA.

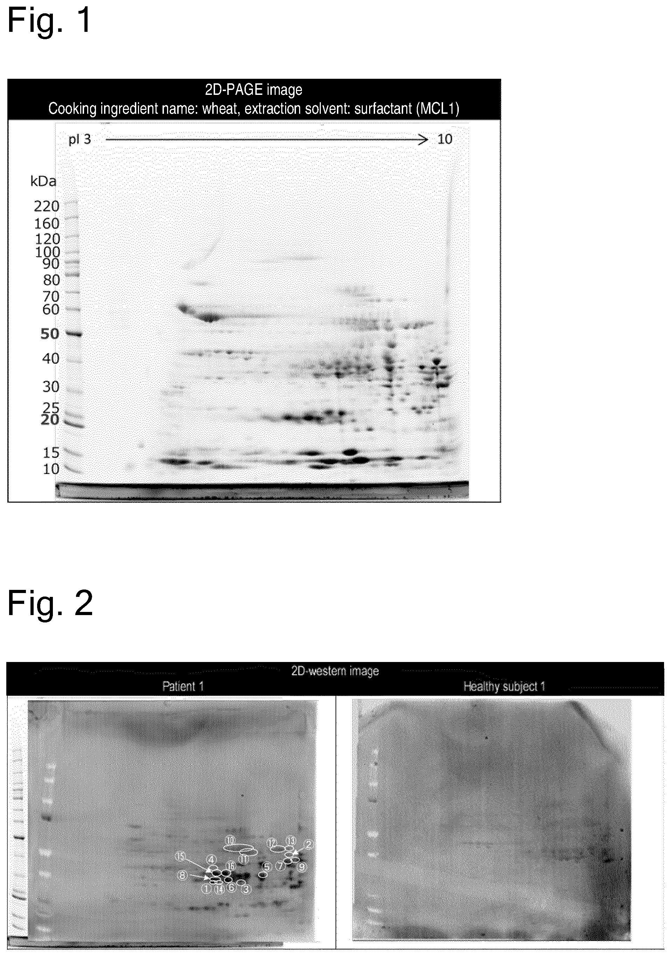

As a result, antigens in the following spots 1 to 16 in a two-dimensional electrophoresis gel run under the conditions described above for proteins in wheat have been revealed to specifically bind to IgE antibodies from wheat-allergic patients (Figs. 2).

### Antigen (protein)

Sequence identification of the antigen in each spot was performed by mass spectrometry. The mass spectroscopic data obtained on a mass spectrometer was analyzed by comparing the data against the Uniprot protein data. As a result, each of spots 1, 7, 11 and 12 was found to be identical to a known sequence.

Information on each spot is summarized in Table 1 below.

**[Table 1-1]**

| | | | MW;35kDa |
|---|---|---|---|
| SPOT No. | | | (1) |
| protein | Isoelectric point | Range | 5-11 |
| | | Preferably | 5.5-10 |
| | | More preferably | 6-9 |
| | Molecular weight | Range | 10-80 |
| | | Preferably | 20-60 |
| | | More preferably | 30-50 |
| | Searched DB | | Uniprot |
| | Organism species | | Wheat (Triticum aestivum) |
| | accession No. | | P18573 |
| | Protein name | | alpha/beta gliadin MM1 |
| | Full-length sequence (SEQ ID NO: 2) | | MKTFLILALLAIVATTARIAVRVPVPQLQPQNPSQQQPQEQVPLVQQQQFPGQQQPFP PQQPYPQPQPFPSQQPYLQLQPFPQPQLPYPQPQLPYPQPQLPYPQPQPFRPQQPYPQS QPQYSQPQQPISQQQQQQQQQQQQKQQQQQQQQILQQILQQQLIPCRDVVLQQHSIA YGSSQVLQQSTYQLVQQLCCQQLWQIPEQSRCQAIHNVVHAIILHQQQQQQQQQQ QPLSQVSFQQPQQQYPSGQGSFQPSQQNPQAQGSVQPQQLPQFEEIRNLALETLPAMC NVYIPPYCTIAPVGIFGTN |
| DNA | Searched DB | | EMBL-EBI |
| | accession No. | | CAA35238.1 |
| | Full-length sequence (SEQ ID NO: 1) | | ATGAAGACCTTTCTCATCCTTGCCCTCCTTGCTATTGTAGCAACCACCGCCAGAATTGCA GTTAGAGTTCCAGTGCCACAATTGCAGCCACAAAATCCATCTCAGCAACAACCACAAGAG CAAGTTCCATTGGTACAACAACAACAATTTCCAGGGCAGCAACAACCATTTCCACCACAA CAGCCATATCCGCAGCCGCAACCATTTCCATCACAACAACCATATCTGCAGCTGCAACCA TTTCCGCAGCCGCAACTACCATATCCGCAGCCGCAACTACCATATCCGCAGCCGCAACTA CCATATCCGCAGCCGCAACCATTTCGACCACAACAACCATATCCACAATCGCAACCACAG TATTCGCAACCACAACAACCAATTTCGCAGCAGCAGCAGCAGCAACAACAACAACAACAA CAAAAACAACAACAACAACAACAACAACAACAGATCCTTCAACAAATTTTGCAACAACAACTG ATTCCATGCAGGGATGTTGTATTGCAACAACACAGCATAGCGTATGGAAGCTCACAAGTT TTGCAACAAAGTACTTACCAGCTGGTGCAACAATTGTGTTGTCAGCAGCTGTGGCAGATC CCCGAGCAGTCGCGGTGCCAAGCCATCCACAATGTTGTTCATGCTATTATTCTGCATCAA CAGCAACAACAACAACAACAACAACAACAACAACCGTTGAGCCAGGTCTCCTTCCAACAG CCTCAACAACAATATCCATCAGGCCAGGGCTCCTTCCAGCCATCTCAGCAAAACCCACAG GCCCAGGGCTCTGTCCAGCCTCAACAACTGCCCCAGTTTGAGGAAATAAGGAACCTAGCG CTAGAGACGCTACCTGCAATGTGCAATGTCTATATCCCTCCATATTGCACCATTGCTCCA GTTGGCATCTTCGGTACTAAC |

**[Table 1-7]**

| | | | MW;45kDa |
|---|---|---|---|
| SPOT No. | | | (7) |
| protein | Isoelectric point | Range | 6-12 |
| | | Preferably | 7-11 |
| | | More preferably | 8-10 |
| | Molecular weight | Range | 20-100 |
| | | Preferably | 25-80 |
| | | More preferably | 30-70 |
| | Searched DB | | Uniprot |
| | Organism species | | Wheat (Triticum aestivum) |
| | accession No. | | M9TG60 |
| | Protein name | | Gamma-gliadin 1 |
| | Full-length sequence (SEQ ID NO: 14) | | MKTLLILTIIAVALTTTTANIQVDPSGQVQWPQQQPFPQPQQPFSQQPQQIFPQPQQT FPHQPQQAFPQPQQTFPHQPQQQFPQPQQPQQPFPQQPQQQFPQPQQPQQPFPQQPQQ QFPQPQQPQQPFPQPQQPQLPFPQQPQQPFPQPQQPQQPFPQLQQPQQPLPQPQQPQQP FPQQQQPLIQPYLQQQMNPCKNYLLQQCNPVSLVSSLVSMILPRSDCKVMRQQCCQQ LAQIPQQLQCAAIHGIVHSIIMQQEQQQQQQQQQQQQQQGIQIMRPLFQLVQGQGII QPQQPAQLEVIRSLVLGTLPTMCNVFVPPECSTTKAPFASIVADIGGQ |
| DNA | Searched DB | | EMBL-EBI |
| | accession No. | | AGJ50340.1 |
| | Full-length sequence (SEQ ID NO: 13) | | ATGAAGACCTTACTCATCCTGACAATCATTGCGGTGGCACTAACTACCACCACCGCCAAT ATACAGGTCGACCCTAGTGGCCAAGTACAATGGCCACAACAACAACAACCATTCCCCCAG CCCCAACAACCATTCTCCCAACAACCACAACAAATTTTTCCCCAACCCCAACAAACATTC CCCCATCAACCACAACAAGCATTTCCCCAACCCCAACAAACATTCCCCCATCAACCACAA CAACAATTTCCCCAGCCCCAGCAACCACAACAACCATTTCCCCAGCAACCACAACAACAA TTTCCCCAGCCCCAACAACCACAACAACCATTTCCCCAGCAACCACAACAACAATTTCCC CAGCCCCAACAACCACAACAACCATTTCCCCAGCCCCAACAACCCCAACTACCATTTCCG CAACAACCACAACAACCATTCCCCCAGCCTCAACAACCCCAACAACCATTTCCCCAGTTA CAGCAACCACAACAACCTTTACCCCAGCCCCAACAACCGCAACAACCATTCCCCCAGCAA CAACAACCATTGATTCAGCCATACCTACAACAACAGATGAACCCCTGCAAGAATTACCTC TTGCAGCAATGCAACCCTGTGTCATTGGTGTCATCCCTCGTGTCAATGATCTTGCCACGA AGTGATTGCAAGGTGATGCGGCAACAATGTTGCCAACAACTAGCACAGATTCCTCAGCAG CTCCAGTGCGCAGCCATCCATGGCATCGTGCATTCCATCATCATGCAGCAAGAACAACAA CAACAACAACAACAACAACAACAACAACAACAACAACAACAAGGCATACAGATCATGCGGCCA CTATTTCAGCTCGTCCAGGGTCAGGGCATCATCCAACCTCAACAACCAGCTCAATTGGAG GTGATCAGGTCATTGGTATTGGGAACTCTTCCAACCATGTGCAACGTGTTTGTTCCACCT GAGTGCTCCACCACCAAGGCACCATTTGCCAGCATAGTCGCCGACATTGGTGGCCAA |

**[Table 1-11]**

| | | | MW;55kDa |
|---|---|---|---|
| SPOT No. | | | (11) (12) |
| protein | Isoelectric point | Range | 5-12 |
| | | Preferably | 5.5-11 |
| | | More preferably | 7-10 |
| | Molecular weight | Range | 20-100 |
| | | Preferably | 25-80 |
| | | More preferably | 30-70 |
| | Searched DB | | Uniprot |
| | Organism species | | Wheat (Triticum aestivum) |
| | accession No. | | B7U6L4 |
| | Protein name | | Globulin 3 |
| | Full-length sequence (SEQ ID NO: 22) | | MATRARVTIPLLFLLGTSLLFAAAVSASHDEEEDRRGGRSLQQCVQRCQQDRPRYSH ARCVQECRDDQQQHGRHEQEEQGRGHGRHGEGEREEEQGRGRGRHGQGEREEEQG RGRGRRGEGERDEEHGDGRRPYVFGPRSFRRIIRSDHGFVKALRPFDEVSRLLRGIRN YRVAIMEVNPRAFVVPGLTDADGVGYVAQGEGVLTVIENGEKRSYTVRQGDVIVAP AGSIMHLANTDGRRKLVIAKILHTISVPGKFQYFSAKPLLASLSKRVLTAALKTSDERL GSLLGSRQGKEEEEKSISIVRASEEQLRELRRQASEGDQGHHWPLPPFRGDSRDTFNL LEQRPKIANRHGRLYEADARSFHALAQHDVRVAVANITPGSMTAPYLNTQSFKLAV VLEGEGEVEIVCPHLGRDSERREQEHGKGRWRSEEEEDDRRQQRRRGSGSESEEEQD QQRYETVRARVSRGSAFVVPPGHPVVEIASSRGSSNLQVVCFEINAERNERVWLAGR NNVIAKLDDPAQELTFGRPAREVQEVFRAKDQQDEGFVAGPEQQQEHERGDRRRGD RGRGDEAVEAFLRMATAAL |
| DNA | Searched DB | | EMBL-EBI |
| | accession No. | | ACJ65514.1 |
| | Full-length sequence (SEQ ID NO: 21) | | ATGGCGACCAGAGCCAGAGTAACCATCCCTCTCCTCTTCCTCCTGGGCACAAGCCTTCTC TTCGCCGCGGCTGTTTCGGCCTCCCATGACGAGGAGGAGGACAGGCGCGGTGGGCGCTCG CTGCAGCAGTGCGTGCAGCGGTGCCAGCAGGACCGGCCGCGGTACTCTCATGCCCGGTGC GTGCAGGAGTGCCGGGACGACCAGCAGCAGCACGGAAGGCACGAGCAGGAGGAGCAGGGC CGCGGGCATGGCCGGCACGGCGAGGGGGAGCGTGAGGAGGAGCAGGGCCGTGGCCGTGGC CGGCACGGCCAGGGAGAGCGTGAGGAGGAGCAGGGCCGTGGACGTGGGCGGCGCGGCGAG GGAGAGCGTGATGAGGAGCACGGGGATGGCCGGCGGCCGTACGTGTTCGGCCCGCGCAGC TTCCGCCGCATCATCCGGAGCGACCACGGGTTCGTCAAGGCCCTTCGCCCGTTCGACGAA GTGTCCAGGCTCCTCCGGGGCATCAGGAACTACCGTGTCGCCATCATGGAGGTGAACCCG CGCGCGTTCGTCGTGCCGGGACTCACGGACGCGGACGGCGTCGGCTACGTCGCTCAAGGC GAGGGGGTGCTGACGGTGATCGAGAACGGCGAGAAGCGGTCCTACACCGTCAGGCAAGGC GATGTGATCGTGGCGCCGGCGGGGTCCATCATGCACCTGGCCAACACCGACGGCCGGAGG AAGCTGGTCATCGCCAAGATTCTCCACACCATCTCCGTCCCCGGCAAGTTCCAGTATTTC TCGGCCAAGCCTCTCCTCGCTAGTTTGAGCAAACGCGTGCTCACAGCGGCGTTAAAGACC TCGGATGAGCGGCTGGGTAGTCTCTTGGGCAGCCGCCAAGGCAAGGAGGAGGAGGAGAAG TCCATCTCCATCGTCCGCGCGTCAGAGGAGCAGCTCCGCGAGCTGCGTCGCCAGGCGTCC GAGGGTGACCAGGGCCACCACTGGCCTCTCCCCCCGTTCCGCGGCGACTCGCGCGACACC TTCAACCTCCTGGAGCAGCGCCCCAAGATCGCCAACCGCCATGGCCGCCTCTACGAGGCC GACGCCCGTAGCTTCCACGCCCTCGCCCAACACGACGTCCGCGTCGCCGTGGCCAACATC ACGCCGGGTTCTATGACCGCACCCTACCTGAACACCCAGTCGTTCAAGCTCGCCGTTGTG CTGGAAGGCGAGGGCGAGGTGGAGATCGTCTGCCCGCACCTCGGCCGCGACAGCGAGCGC CGCGAGCAAGAGCACGGCAAGGGCAGGTGGAGGAGCGAGGAAGAGGAGGACGACCGGCGG CAGCAACGCCGACGCGGGTCCGGCTCCGAGTCGGAGGAGGAGCAGGACCAGCAGAGGTAC GAGACGGTCCGCGCGCGGGTGTCGCGCGGCTCGGCGTTCGTGGTGCCCCCCGGCCACCCG GTGGTGGAGATCGCCTCGTCCCGCGGCAGCAGCAACCTCCAGGTGGTGTGCTTCGAGATC AACGCCGAGAGGAACGAGCGGGTGTGGCTCGCCGGGAGGAACAACGTGATCGCCAAGCTG GACGACCCCGCCCAGGAGCTCACCTTCGGCAGGCCCGCGAGGGAGGTGCAGGAGGTGTTC CGCGCCAAGGATCAGCAGGACGAGGGCTTCGTCGCCGGACCCGAGCAGCAGCAGGAGCAT GAGCGCGGGGACCGCCGCCGTGGTGACCGCGGGCGCGGCGACGAAGCCGTGGAGGCGTTC CTGAGGATGGCAACCGCCGCGCTC |

Spots 11 and 12 are derived from the same protein, and a total of 3 types of proteins (1), (7) and (11) were identified as novel antigens derived from wheat.

The antigen in spot 1 in the present invention is not limited and can be any of the proteins as defined below in (1-a) to (1-f).
(1-a) a protein comprising the amino acid sequence of SEQ ID NO: 2;
   (1-b) a protein comprising an amino acid sequence with deletion, substitution, insertion or addition of one or several amino acids in SEQ ID NO: 2;
   (1-c) a protein comprising an amino acid sequence having at least 70% identity to the amino acid sequence of SEQ ID NO: 2;
   (1-d) a protein comprising an amino acid sequence encoded by a nucleotide sequence with deletion, substitution, insertion or addition of one or several nucleotides in SEQ ID NO: 1;
   (1-e) a protein comprising an amino acid sequence encoded by a nucleotide sequence having at least 70% identity to the nucleotide sequence of SEQ ID NO: 1; and
   (1-f) a protein comprising an amino acid sequence encoded by a nucleic acid that hybridizes under stringent conditions with a nucleic acid having a nucleotide sequence complementary to the nucleotide sequence of SEQ ID NO: 1.
(7-a) a protein comprising the amino acid sequence of SEQ ID NO: 14;
   (7-b) a protein comprising an amino acid sequence with deletion, substitution, insertion or addition of one or several amino acids in SEQ ID NO: 14;
   (7-c) a protein comprising an amino acid sequence having at least 70% identity to the amino acid sequence of SEQ ID NO: 14;
   (7-d) a protein comprising an amino acid sequence encoded by a nucleotide sequence with deletion, substitution, insertion or addition of one or several nucleotides in SEQ ID NO: 13;
   (7-e) a protein comprising an amino acid sequence encoded by a nucleotide sequence having at least 70% identity to the nucleotide sequence of SEQ ID NO: 13; and
   (7-f) a protein comprising an amino acid sequence encoded by a nucleic acid that hybridizes under stringent conditions with a nucleic acid having a nucleotide sequence complementary to the nucleotide sequence of SEQ ID NO: 13.
(11-a) a protein comprising the amino acid sequence of SEQ ID NO: 22;
   (11-b) a protein comprising an amino acid sequence with deletion, substitution, insertion or addition of one or several amino acids in SEQ ID NO: 22;
   (11-c) a protein comprising an amino acid sequence having at least 70% identity to the amino acid sequence of SEQ ID NO: 22;
   (11-d) a protein comprising an amino acid sequence encoded by a nucleotide sequence with deletion, substitution, insertion or addition of one or several nucleotides in SEQ ID NO: 21;
   (11-e) a protein comprising an amino acid sequence encoded by a nucleotide sequence having at least 70% identity to the nucleotide sequence of SEQ ID NO: 21; and
   (11-f) a protein comprising an amino acid sequence encoded by a nucleic acid that hybridizes under stringent conditions with a nucleic acid having a nucleotide sequence complementary to the nucleotide sequence of SEQ ID NO: 21.

The proteins that are the aforementioned antigens (1), (7) and (11) and polypeptides (E4), (E9), (E11), (E19), (E31), (E36), (E37), (E43) and (E44) mentioned later include those proteins or polypeptides in a form whose amino acid residues are modified by phosphorylation, sugar chain modification, aminoacylation, ring-opening, deamination or the like.

The proteins that are the aforementioned antigens (1), (7) and (11) and polypeptides (E4), (E9), (E11), (E19), (E31), (E36), (E37), (E43) and (E44) mentioned later are preferably antigens of an allergy.

By stating herein "deletion, substitution, insertion or addition of one or several amino acids" in relation to amino acid sequence, it is meant that in an amino acid sequence of interest, one or several amino acids are deleted, one or several amino acids are substituted by any other amino acids, any other amino acids are inserted, and/or any other amino acids are added. "Several amino acids" are not limited and mean at least 200, at least 100, at least 50, at least 30, at least 20, at least 15, at least 12, at least 10, at least 8, at least 6, at least 4 or at least 3 amino acids. Alternatively, several amino acids mean 30%, preferably 25%, 20%, 15%, 10%, 5%, 3%, 2% or 1%, of amino acids with respect to the total length of the amino acid sequence.

Among the aforementioned modifications, substitution is preferably conservative substitution. The "conservative substitution" refers to the substitution of a certain amino acid residue by a different amino acid residue having similar physicochemical characteristics, and can be any type of substitution as long as it does not substantially change the characteristics of the structure of the original sequence for example, any type of substitution is acceptable as long as any substituted amino acids do not disrupt the helical structure of the original sequence or other secondary structures that characterize the original sequence. The following gives examples of separate groups of amino acid residues that are conservatively substitutable with each other, but substitutable amino acid residues are not limited to the examples given below.
Group A: leucine, isoleucine, valine, alanine, methionine, glycine, cysteine, proline
Group B: aspartic acid, glutamic acid
Group C: asparagine, glutamine
Group D: lysine, arginine
Group E: serine, threonine
Group F: phenylalanine, tyrosine, tryptophan, histidine

In the case of non-conservative substitution, one member belonging to one of the aforementioned groups can be replaced with a member belong to any other group. For example, in order to eliminate the possibility of unwanted sugar-chain modification, amino acids of group B, D or E as listed above may be substituted by those of any other group. Also, cysteines may be deleted or substituted by any other amino acids to prevent them from being folded into a protein in its tertiary structure. Also, in order to maintain the balance between hydrophilicity and hydrophobicity or to increase hydrophilicity for the purpose of facilitating synthesis, any amino acids may be substituted in consideration of the hydropathy scales of amino acids, which are a measure of the hydrophilic and hydrophobic properties of amino acids (J. Kyte and R. Doolittle, J. Mol. Biol., Vol.157, p.105-132, 1982).

In another embodiment, substitution of the original amino acid by an amino acid with less steric hindrance, for example, substitution of group F by group A, B, C, D or E; or substitution of an amino acid having an electric charge by an amino acid having no electric charge, for example, substitution of group B by group C, may be performed. This may improve binding activity against IgE antibodies.

As referred to herein, the percent identity between two amino acid sequences can be determined by visual inspection and mathematical calculation. Alternatively, the percent identity can be determined using a computer program. Examples of such computer programs include BLAST and ClustalW. In particular, various conditions (parameters) for identity searches with the BLAST program are described in Altschul, et al. (Nucl. Acids. Res., 25, p.3389-3402, 1997), and are publicly available on the websites of the NCBI and DNA Data Bank of Japan (DDBJ) (Altschul, et al., BLAST Manial, Altschul, et al., NCB/NLM/NIH Bethesda, MD 20894). Also, the percent identity can be determined using a genetic information processing software program, such as GENETYX Ver.7 (Genetyx Corporation), DINASIS Pro (Hitachi Software Engineering Co., Ltd.), or Vector NTI (Infomax Inc.).

By stating herein "deletion, substitution, insertion or addition of one or several nucleotides" in relation to nucleotide sequence, it is meant that in a nucleotide sequence of interest, one or several nucleotides are deleted, one or several nucleotides are substituted by any other nucleotides, any other nucleotides are inserted, and/or any other nucleotides are added. "Several nucleotides" are not limited and mean at least 600, at least 300, at least 150, at least 100, at least 50, at least 30, at least 20, at least 15, at least 12, at least 10, at least 8, at least 6, at least 4 or at least 3 nucleotide acids. Alternatively, several nucleotides mean 30%, preferably 25%, 20%, 15%, 10%, 5%, 3%, 2% or 1%, of nucleotides with respect to the total length of the nucleotide sequence. It is preferable that such a nucleotide deletion, substitution, insertion or addition should not give rise to a frame shift in an amino acid coding sequence.

As referred to herein, the percent identity between two nucleotide sequences can be determined by visual inspection and mathematical calculation. Alternatively, the percent identity can be determined using a computer program. Examples of such sequence comparison computer programs include the BLASTN program, version 2.2.7, available on the website of the National Library of Medicine: https://blast.ncbi.nlm.nih.gov/Blast.cgi (Altschul, et al. (1990) J. Mol. Biol., 215: 403-10), or the WU-BLAST 2.0 algorithm. Standard default parameter settings for WU-BLAST 2.0 are found and available on the following website: http://blast.wustl.edu.

As referred to herein, "under stringent conditions" means that hybridization takes place under moderately or highly stringent conditions. To be specific, the moderately stringent conditions can be easily determined by those having ordinary skill in the art on the basis of, for example, the length of DNA. Basic conditions are described in Sambrook, et al., Molecular Cloning: A Laboratory Manual, 3rd ed., ch.6-7, Cold Spring Harbor Laboratory Press, 2001. The moderately stringent conditions include hybridization under the conditions of preferably 1×SSC to 6xSSC at 42°C to 55°C, more preferably 1×SSC to 3×SSC at 45°C to 50°C, most preferably 2×SSC at 50°C. In the case of using a hybridization solution containing, for example, about 50% formamide, a temperature around 5 to 15°C lower than the foregoing should be adopted. Washing is also carried out under the conditions of 0.5xSSC to 6×SSC at 40°C to 60°C. In the process of hybridization and washing, generally 0.05% to 0.2% SDS, preferably about 0.1% SDS, may be added. Likewise, the highly stringent conditions can be easily determined by those having ordinary skill in the art on the basis of, for example, the length of DNA. Generally, the highly stringent (high stringent) conditions include hybridization and/or washing at a higher temperature and/or a lower salt concentration than those adopted under the moderately stringent conditions. For example, hybridization is carried out under the conditions of 0.1×SSC to 2×SSC at 55°C to 65°C, more preferably 0.1×SSC to 1×SSC at 60°C to 65°C, most preferably 0.2xSSC at 63°C. Washing is carried out under the conditions of 0.2×SSC to 2×SSC at 50°C to 68°C, more preferably 0.2xSSC at 60 to 65°C.

Variants corresponding to the proteins as defined above in (b) to (f) of (1), (7) and (11) are not limited and preferably comprise an amino acid sequence of an epitope (variant) mentioned later. The amino acid sequence of the epitope contained is not limited to one sequence and preferably includes all sequences of epitopes derived from each protein. For example, epitopes derived from the protein as defined in (1) are (E9), (E31), (E11), (E36) and (E43). Thus, the variants as defined in (1-b) to (1-f) preferably comprise one or more of the amino acid sequences (including their variants) of the epitopes (E9), (E31), (E11), (E36) and (E43).

Antigens may be obtained by separating and purifying them from wheat using a combination of protein purification methods well known to those skilled in the art. Also, antigens may be obtained by expressing them as recombinant proteins using a genetic recombination technique well known to those skilled in the art and by separating and purifying them using protein purification methods well known to those skilled in the art.

Exemplary protein purification methods include: solubility-based purification methods such as salt precipitation and solvent precipitation; purification methods based on molecular weight difference, such as dialysis, ultrafiltration, gel filtration and SDS-PAGE; charge-based purification methods such as ion exchange chromatography and hydroxylapatite chromatography; specific affinity-based purification methods such as affinity chromatography; purification methods based on hydrophobicity difference, such as reverse-phase high-performance liquid chromatography; and purification methods based on isoelectric point difference, such as isoelectric focusing.

Preparation of a protein by a genetic recombination technique is carried out by preparing an expression vector comprising an antigen-encoding nucleic acid, introducing the expression vector into appropriate host cells by gene transfer or genetic transformation, culturing the host cells under suitable conditions for expression of a recombinant protein, and recovering the recombinant protein expressed in the host cells.

The "vector" refers to a nucleic acid that can be used to introduce a nucleic acid attached thereto into host cells. The "expression vector" is a vector that can induce the expression of a protein encoded by a nucleic acid introduced therethrough. Exemplary vectors include plasmid vectors and viral vectors. Those skilled in the art can select an appropriate expression vector for the expression of a recombinant protein depending on the type of host cells to be used.

The "host cells" refers to cells that undergo gene transfer or genetic transformation by a vector. The host cells can be appropriately selected by those skilled in the art depending on the type of the vector to be used. The host cells can be derived from prokaryotes such as E. coli. When prokaryotic cells like E coli. are used as host cells, the antigen of the present invention may be designed to contain an N-terminal methionine residue in order to facilitate the expression of a recombinant protein in the prokaryotic cells. The N-terminal methionine can be cleaved from the recombinant protein after expression. Also, the host cells may be cells derived from eukaryotes, such as single-cell eukaryotes like yeast, plant cells and animal cells (e.g., human cells, monkey cells, hamster cells, rat cells, murine cells or insect cells) or silkworm.

Gene transfer or genetic transformation of an expression vector into host cells can be carried out as appropriate by following a technique known to those skilled in the art. Those skilled in the art can make possible the expression of a recombinant protein by selecting suitable conditions for the expression of the recombinant protein as appropriate depending on the type of host cells and culturing the host cells under the selected conditions. Then, those skilled in the art can homogenize the host cells having the expressed recombinant protein, and separate and purify an antigen expressed as the recombinant protein from the resulting homogenate by using an appropriate combination of such protein purification methods as mentioned above. The aforementioned expression vector or synthesized double-stranded DNA, or mRNA transcribed therefrom is introduced into a cell-free protein synthesis system for expression, and the expressed protein can be separated and purified to prepare an antigen.

Preferably, the antigen of the present invention specifically binds to an IgE antibody from an allergic patient.

### Diagnosis kit and method (1)

The present invention provides a method for providing an indicator for diagnosing an allergy in a subject, the method comprising the steps of:
(i) contacting a sample obtained from the subject with an antigen, wherein the sample is a solution comprising an IgE antibody;
(ii) detecting binding between an IgE antibody present in the sample from the subject and the antigen; and
(iii) when the binding between the IgE antibody in the subject and the antigen is detected, an indicator of the fact that the subject is allergic is provided;
wherein the antigen is at least one of proteins as defined above in any of (1), (7) and (11).

In one embedment, the "allergy" is not limited and is an allergy to grain, preferably an allergy to grain of the family *Poaceae* or an allergy to wheat.

As referred to herein, the "diagnosis" includes mere "detection" having a potential, in addition to (definitive) diagnosis that is generally made by a physician. As referred to herein, in one embodiment, the "diagnosis" and the "detection" are *in vivo, in vitro* or *ex vivo* "diagnosis" and "detection". *In vitro* "diagnosis" and "detection" are preferred.

The sample obtained from a subject is a solution containing an IgE antibody, as collected from the subject. Examples of such solutions include blood, saliva, sputum, snivel, urine, sweat, and tear. The sample obtained from the subject may be subjected to pretreatment for increasing the concentration of an IgE antibody in the sample before being contacted with an antigen. The pretreatment of a sample may involve, for example, collection of the serum or the plasma from the blood. Furthermore, a Fab moiety that is an antigen-binding moiety may be purified. In a particularly preferred embodiment, the step (i) mentioned above is carried out by contacting an IgE antibody present in the serum obtained from a subject with an antigen.

The IgE antibody may be the IgE antibody itself or may be mast cells or the like bound to IgE antibodies.

Detection of contact and binding between the sample obtained from a subject and an antigen can be carried out by using a known method. Examples of such methods that can be used include detection by ELISA (Enzyme-Linked Immunosorbent Assay), sandwich immunoassay, immunoblotting technique, immunoprecipitation, or immunochromatography. These are all techniques for detecting binding between an antigen and an IgE antibody from a subject by contacting and binding the IgE antibody from a subject with the antigen, allowing an enzymatically labelled secondary antibody to act on the IgE antibody specifically bound to the antigen, and adding an enzyme substrate (generally, chromogenic or luminescent reagent) to detect an enzymatic reaction product. Also, a method for detecting a fluorescently labeled secondary antibody can be used. Alternatively, detection by a measurement method capable of evaluating binding between an antigen and an IgE antibody, such as surface plasmon resonance (SPR), can also be used. A plurality of antigen-specific IgE antibodies may be mixed.

The antigen may be provided as an isolated antigen in a state immobilized on a carrier. In this case, the steps (i) and (ii) mentioned above can be carried out using ELISA, sandwich immunoassay, immunochromatography, surface plasmon resonance, or the like. Also, the step (i) mentioned above can be carried out by contacting the sample obtained from a subject with an antigen-immobilized surface. The isolated antigen may be obtained by separating and purifying it from a subject (raw materials, processed products, etc.) using a combination of protein purification methods well known to those skilled in the art, or by preparing it using a genetic recombination technique. Alternatively, an antibody may be attached to a carrier.

The antigen may be in a state unimmobilized on a carrier. In this case, flow cytometry or the like can be used in the aforementioned steps (i) and (ii), and the presence of the antigen bound to the antibody can be confirmed with laser beam. Examples include a basophil activation test (BAT). Another example includes a histamine release test (HRT) which examines whether histamine is released by further contacting an antigen with blood cells in a sample.

The antigen may be detected by an immunoblotting technique after transfer from a state separated by two-dimensional electrophoresis. The two-dimensional electrophoresis is a technique for separating a protein sample by performing isoelectric focusing in the first dimension and performing SDS-PAGE in the second dimension. The conditions for two-dimensional electrophoresis are not particularly limited as long as the conditions permit the separation of the antigen of the present invention. For example, the conditions for two-dimensional electrophoresis as described above in the subsection titled "Identification of antigens" can be adopted. Also, the electrophoresis conditions may be defined by reference to the descriptions in Patent Literatures 1 to 4 mentioned above. For example, two-dimensional electrophoresis can be carried out under the conditions that satisfy at least one selected from the group consisting of the following requirements:
(A) the isoelectric focusing gels used in the first dimension should have a gel-strip length of 5 to 10 cm and a gel pH range of 3 to 10, and the pH gradient of the gels in the direction of electrophoresis should be as follows: where the gel-strip length up to pH 5 is taken as "a", that length from pH 5 to 7 as "b", and that length above pH 7 as "c", the relations "a < b" and "b > c" are satisfied;
(B) in the case of (A), when the total gel-strip length is taken as 1, "a" should be in the range of 0.15 to 0.3, "b" should be in the range of 0.4 to 0.7, and "c" should be in the range of 0.15 to 0.3;
(C) in the first dimensional isoelectric focusing, a constant voltage step should be performed by applying a constant voltage ranging from 100 V to 600 V per gel strip containing a sample, and after the electrophoresis variation width during electrophoresis for 30 minutes falls within the range of 5 µA, a voltage-increasing step should be started at which the voltage is increased from the aforementioned constant voltage;
(D) in the case of (C), the final voltage at the voltage-increasing step should be in the range of 3000 V to 6000 V;
(E) the isoelectric focusing gels used in the first dimension should have a longitudinal gel-strip length of 5 to 10 cm, and the electrophoresis gels used in the second dimension should have a gel concentration at the distal end in the direction of electrophoresis, which is in the range of 3 to 6%; and
(F) in the case of (E), the electrophoresis gels used in the second dimension should have a gel concentration at the proximal end in the direction of electrophoresis, which is set to a higher value than that at the distal end in the direction of electrophoresis.

The aforementioned antigens (1), (7) and (11) are antigens that specifically bind to IgE antibodies from allergic patients (e.g., patients with allergy to wheat). Therefore, when binding between an IgE antibody from a subject and the antigen is detected, an indicator of the fact that the subject is allergic is provided.

The present invention further provides a kit for diagnosing an allergy, comprising at least one of the aforementioned antigens (1), (7) and (11). The diagnosis kit of this invention may be used in the aforementioned method for providing an indicator for diagnosing an allergy or in a diagnosis method as described later. The diagnosis kit of this invention may comprise not only the at least one of the aforementioned antigens (1), (7) and (11), but also an anti-IgE antibody labeled with an enzyme and a chromogenic or luminescent substrate serving as a substrate for the enzyme. Also, a fluorescent-labeled anti-IgE antibody may be used. In the diagnosis kit of this invention, the antigen may be provided in a state immobilized on a carrier. The diagnosis kit of this invention may also be provided together with instructions on the procedure for diagnosis or a package containing said instructions.

In another embodiment, the aforementioned diagnosis kit comprises a companion diagnostic agent for an allergy. The companion diagnostic agent is used for identifying patients expected to respond to pharmaceutical products or identifying patients having the risk of severe adverse reactions to pharmaceutical products, or for studying the reactivity of pharmaceutical products in order to optimize treatment using the pharmaceutical products. Here, the optimization of treatment includes, for example, determination of dosage and administration, judgment regarding discontinuation of administration, and confirmation of an allergen component that is used to cause immunological tolerance.

The present invention further provides a composition for diagnosing an allergy, comprising at least one of the aforementioned antigens (1), (7) and (11). The diagnosis composition of this invention can be used in a diagnosis method as described below. The diagnosis composition of this invention may further comprise a pharmaceutically acceptable carrier and/or additives commonly used with the antigen of this invention depending on the need.

In one embodiment, the present invention provides a method for diagnosing an allergy in a subject, the method comprising:
(i) contacting a sample obtained from the subject with an antigen;
(ii) detecting binding between an IgE antibody present in the sample from the subject and the antigen; and
(iii) when the binding between the IgE antibody in the subject and the antigen is detected, diagnosing the subject as being allergic;
wherein the antigen is at least one of proteins as defined above in any of (1), (7) and (11). In this method, the steps (i) and (ii) are performed as described above regarding the corresponding steps of the method for providing an indicator for diagnosing an allergy.

In another embodiment, the present invention provides a method for diagnosing an allergy in a subject, the method comprising administering to the subject at least one of the aforementioned antigens (1), (7) and (11). This method may be performed in the form of a skin test characterized by applying the antigen onto the skin. Examples of the skin test include various forms of tests, such as: a prick test in which a diagnosis composition is applied onto the skin and then a tiny prick to such an extent as not to provoke bleeding is made in the skin to allow an antigen to penetrate the skin, thereby observing a skin reaction; a scratch test in which a diagnosis composition is applied onto the skin and then the skin is lightly scratched to observe a reaction; a patch test in which a diagnosis composition in the form of cream, ointment, etc. is applied onto the skin to observe a reaction; and an intracutaneous test in which an antigen is administered intracutaneously to observe a reaction. If a skin reaction such as swelling occurs in a skin portion to which the antigen has been applied, the subject of interest is diagnosed as having an allergy. The amount of the antigen to be applied to the skin in such tests can be, for example, not more than 100 µg per dose.

In the process of allergy diagnosis, a load test aiming to identify an antigen is often adopted. At least one of the aforementioned antigens (1), (7) and (11) can be used as an active ingredient for a load test to diagnose an allergy. Here, the antigen protein to be used in the load test may be a protein that has been expressed and purified and may be a protein that has been expressed in raw materials or processed products, such as rice-based vaccine expressing pollen allergens which are obtained by transforming rice with a gene of a cedar pollen antigen and expressing the antigen protein in the rice.

In one embodiment, the diagnosis composition or the diagnosis kit described above may be used for a prick test, a scratch test, a patch test, an intracutaneous test or the like.

In yet another embodiment, the present invention provides at least one of the aforementioned antigens (1), (7) and (11), intended for use in the diagnosis of an allergy. This also includes the provision of at least one of the aforementioned antigens (1), (7) and (11) mixed with a known antigen.

In still another embodiment, the present invention provides use of at least one of the aforementioned antigens (1), (7) and (11) for the production of a composition for diagnosing an allergy.

### Composition and treatment method (1)

The present invention provides a composition comprising at least one of the aforementioned antigens (1), (7) and (11).

In one embodiment, the composition of the present invention is a pharmaceutical composition. In one embodiment, the composition of the present invention is a quasi-pharmaceutical composition or a nonpharmaceutical composition (e.g., a cosmetic composition and a food composition).

In one embodiment, the aforementioned composition is used for the treatment of an allergy (e.g., an allergy to wheat). As referred to herein, the "treatment of an allergy" increases the limit amount of an antigen in which the allergy does not develop even if the antigen is incorporated into the body, and finally aims for the state where the allergy does not develop by the common amount of the antigen to be consumed (remission).

The present invention also provides a method for treating an allergy, the method comprising administering at least one of the aforementioned antigens (1), (7) and (11) to a patient in need of a treatment for an allergy.

In another embodiment, the present invention provides at least one of the aforementioned antigens (1), (7) and (11), intended for use in the treatment for an allergy. In yet another embodiment, the present invention provides use of at least one of the aforementioned antigens (1), (7) and (11) for the production of a therapeutic agent for an allergy.

In the process of allergy treatment, a hyposensitization therapy aiming to induce immunological tolerance by administering an antigen to a patient is often adopted. The at least one of the aforementioned antigens (1), (7) and (11) can be used as an active ingredient for a hyposensitization therapy for an allergy. Here, the antigen protein to be used in the hyposensitization therapy may be a protein that has been expressed and purified and may be a protein that has been expressed in raw materials or processed products, such as rice-based vaccine expressing pollen allergens which are obtained by transforming rice with a gene of a cedar pollen antigen and expressing the antigen protein in the rice.

The composition of the present invention can be administered by common administration routes. Examples of common administration routes include oral, sublingual, percutaneous, intracutaneous, subcutaneous, intravascular, intranasal, intramuscular, intraperitoneal, and intrarectal administrations.

The composition of the present invention can be used as a composition to which a commonly used pharmaceutically acceptable adjuvant or excipient or any other additives (e.g., stabilizer, solubilizer, emulsifier, buffer, preservative, colorant) are added by a conventional method together with the antigen of this invention depending on the need. The dosage form of the composition can be selected by those skilled in the art as appropriate depending on the administration route. The composition can be in the form of, for example, tablet, capsule, troche, sublingual tablet, parenteral injection, intranasal spray, poultice, solution, cream, lotion, or suppository. The administration dose, frequency and/or period of the composition of this invention can be selected by a physician as appropriate depending on the administration route and the patient's condition and characteristics such as age and body weight. For example, the composition may be administered to an adult patient at a dose of not more than 100 µg per dose. The administration interval can be, for example, once daily, once weekly, twice weekly, once per three months or so. The administration period can be, for example, several weeks to several years. The composition may be administered in such a manner that the dose is increased in incremental steps over the administration period.

### Tester Composition (1)

The present invention provides a tester composition comprising an antibody for at least one of the aforementioned antigens (1), (7) and (11).

The antibody can be prepared by a conventional method. For example, the antibody may be prepared by immunizing a mammal such as rabbit with one of the aforementioned antigens (1), (7) and (11). The antibody may be an Ig antibody, a polyclonal antibody, a monoclonal antibody, or an antigen-binding fragment thereof (e.g., Fab, F(ab')₂, Fab').

Further, in the aforementioned tester composition, the antibody may be provided in a form bound to a carrier. The type of the carrier is not particularly limited as long as it is usable for detection of binding between an antibody and an antigen. Any given carrier known to those skilled in the art can be used.

Examples of a method for determining the presence or absence of an antigen include the following methods:
a method in which a prepared tester composition comprising an Ig antibody is contacted with a sample obtained from raw materials or processed products, etc., ELISA or the like method is used to detect whether there is a binding between the Ig antibody and an antigen in the sample, and if the binding between the Ig antibody and the antigen is detected, it is determined that the antigen is contained in the raw materials or processed products, etc. of interest; and
a method in which filter paper is impregnated with raw materials or processed products and reacted with an antibody solution so as to detect an antigen contained therein.

Another embodiment of the present invention includes a tester composition for determining the presence or absence of an antigen of an allergy in an object of interest, the tester composition comprising a primer having a nucleotide sequence complementary to a portion of at least one of the nucleotide sequences of SEQ ID NOs: 1, 13 or 21. The primer has, for example, but not limited to, a nucleotide sequence complementary to, preferably, 12 bases, 15 bases, 20 bases, or 25 bases, in a 3'-terminal or central sequence in a partial sequence of at least one of the nucleotide sequences of SEQ ID NOs: 1, 13 or 21. Particularly, when mRNA is of interest, the tester has a complementary primer of a poly-A tail. In a preferred embodiment, the tester composition comprising the primer mentioned above may further comprise a primer comprising a 5'-terminal nucleotide sequence, preferably a nucleotide sequence of 12 bases, 15 bases, 20 bases, or 25 bases, of at least one of the nucleotide sequences of SEQ ID NOs: 1, 13 or 21.

For example, cDNA is amplified by PCR (Polymerase Chain Reaction) including RT-PCR (Reverse Transcription-Polymerase Chain Reaction) using templated DNA or mRNA obtained from wheat and the aforementioned complementary primer, and the sequence of the amplified cDNA is compared with SEQ ID NOs: 1, 13 or 21 to determine the presence or absence of the antigen. Amplification methods by PCR can be exemplified by RACE (Rapid Amplification of cDNA End). In this respect, even if there exists a point mutation encoding the same amino acid in the comparison of the amplified cDNA with SEQ ID NOs: 1, 13 or 21, or even if the nucleotide sequence of the amplified cDNA has insertion, deletion, substitution or addition of bases in the nucleotide sequences of SEQ ID NOs: 1, 13 or 21, it is determined that the antigen is present when the amino acid sequence encoded by the cDNA has at least 70%, preferably at least 80, 90, 95, 98, or 99% identity to the amino acid sequence of SEQ ID NOs: 2, 14 or 22.

In one embodiment, the aforementioned tester composition, for example, is used to determine the presence or absence of an antigen in cooking ingredients (wheat) or in products of interest in a food production line. The tester composition may also be used for quality inspection of production lines and pre-shipment products by manufacturers, or may be used for self-checking of the presence or absence of an antigen in raw materials or processed products of interest by consumers. Also, it may be used for checking of the presence or absence of an antigen based on increase or decrease in the peak of the protein using a mass spectrometer.

### Method for determining presence or absence of antigen (1)

The present invention includes a method for determining the presence or absence of at least one of the aforementioned antigens (1), (7) and (11) in a substance of interest, comprising contacting an antibody for the at least one of the aforementioned antigens (1), (7) and (11) with a raw material or a processed product (including a liquid).

The raw material may be a cooking ingredient or may be a cosmetic raw material, a pharmaceutical raw material or the like. The processed product may be an edible processed product or may be a cosmetic, a pharmaceutical product or the like.

Such an antibody, a method for preparing the antibody, a method for contacting the antibody with a raw material or a processed product, the binding between the antibody and the antigen, etc. are as described above in the subsection titled "Tester composition (1)".

### Antigen-free food and the like (1)

The present invention provides a raw material or processed product in which at least one of the aforementioned antigens (1), (7) and (11) is eliminated or reduced. In one embodiment, the "raw material or processed product" is "wheat or a processed product of wheat".

The method for eliminating or reducing the antigen of the present invention in a raw material or processed products is not limited. The elimination or reduction of the antigen may be conducted by any method, as long as the method permits the elimination or reduction of the antigen.

For example, the raw material (e.g., wheat) of the present invention whose antigen is eliminated or reduced may be obtained by preparing a raw material in which the expression of the antigen of the present invention is modified, using a gene modification technique.

Any technique known to those skilled in the art can be used as the genetic modification technique. For example, Oishi, et al. (Scientific Reports, Vol. 6, Article number: 23980, 2016, doi:10.1038/srep23980) states that individuals with ovomucoid gene deletion are obtained by applying a genome editing technique CRISPER/Cas9 to chicken primordial germ cells. The raw material of the present invention whose antigen is eliminated may be obtained through the use of a similar technique.

The wheat of the present invention whose antigen is reduced may be obtained by mating through artificial insemination with raw materials (e.g., wheat) containing no antigen or containing the antigen in a small amount. The artificial crossing of raw materials can be performed by a conventional method, for example, a method for pollinating selected breeds for the purpose of breed improvement of foods. For example, hypoallergenic wheat 1BS-18 Hokushin can be selected as antigen-deficient wheat for the selected breeds.

An antigen of the present invention may be the artefact that an antigen of the present invention assumed the removal or a reduced raw material as for the removal or the reduced processed products. In the case of using an ordinary raw material as a material, a treatment for removing or reducing the antigen of this invention is performed before or after preparation of a processed product. The methods for removing or reducing the antigen of the present invention in the processed products which assumed an ordinary raw material as a material include a method to remove protein component in raw materials or processed products such as a high pressure treatment and elution with the neutral salt solution or the high temperature steam, and a method to perform hydrolysis, denaturation, or amino acid alteration (chemical modification, elimination, or the like of a side chain) by heat treatment and acid treatment.

### Method for producing raw material or processed product in which antigen is eliminated or reduced (1)

The present invention provides a method for producing a raw material or processed products in which an antigen is eliminated or reduced, the method comprising the step of confirming that the antigen is eliminated or reduced, in a production process of the processed product, wherein the antigen is at least one of the aforementioned antigens (1), (7) and (11).

The step of confirming that the antigen is eliminated or reduced, in a production process of the raw materials or processed products in which an antigen is eliminated or reduced may be performed by confirming the presence or absence of an antigen by the method described above in the subsection titled "Tester Composition (1)".

The production of the wheat or processed products of wheat in which an antigen is eliminated or reduced may be performed by the method described above in the subsection titled "Antigen-free food and the like".

### Epitope

Epitopes and amino acids important for binding activity against IgE antibodies from allergic patients within the epitopes were identified as shown in Example 4 as to antigens identified as shown in Examples 1-3.

The results are summarized in Table 2.

**[Table 2-6]**

| E4 | | | | | | | |
|---|---|---|---|---|---|---|---|
| Spot No. 7 | | | | | | | |
| Protein name | Gamma gliadin-A1 M9TG60 | | SEQ ID NO | Food in which cross-reactivity was confirmed | Graph No. | Synthetic sequence | SEQ ID NO |
| 15-residue seauence | QQPQQPFPQQQQPLI (positions 171-185 of SEQ ID NO: 14) | | 281 | | | | |
| P2 | More preferably | QQPQQXXPQQXXXXX | 282 | | | | |
| | Preferably | QXPQQXXPQQXXXXX | 283 | | | | |
| | Key | QXXQQXXPQQXXXXX | 284 | | | | |
| | Further preferably | QPFPQQQQP | 285 | | | | |
| | More preferably | QPFPQQQQ | 286 | | | | |
| | Preferably | QPFPQQQ | 287 | | | | |
| | Key | QXXPQQX | 288 | | | | |
| P4 | Key | XXPQQXFPQQXXXXX | 289 | | | | |
| | More preferably | QQPFPQQQ | 290 | | | | |
| | Preferably | QPFPQQ | 291 | | | | |
| | Key | QXFPQQ | 292 | | | | |
| P7 | Preferably | QPQQPQQP | 293 | | | | |
| | Key | QPQQP | 294 | | | | |
| | Preferably | QPFPQQQQPL | 295 | | | | |
| | Key | QPFPQQQQP | 296 | | | | |
| P12 | Key | QPFPQQQ | 297 | | | | |
| P14 | Key | XXXQQXXPQQQXXXX | 298 | | | | |
| | Key | QPFPQQQ | 299 | | | | |
| P15 | Key | XXPQQXXPQQQXXXX | 300 | | | | |
| | More preferably | QPFPQQQQ | 301 | | | | |
| | Preferably | PFPQQQ | 302 | | | | |
| | Key | XXPQQQX | 303 | | | | |
| P19 | Preferably | XQPQQXXPQQXXXLI | 304 | | | | |
| | Key | XQPQQXXPQQXXXXX | 305 | | | | |
| | Key | QPFPQQQQ | 306 | | | | |
| P21 | Preferably | QQPFPQQ | 307 | | | | |
| | Key | QPFPQQ | 308 | | | | |
| P22 | Key | XQPQQXXPXQQXPXX | 309 | | | | |
| | Preferably | QPFPQQQQP | 310 | | | | |
| | Key | QPFPQQQ | 311 | | | | |
| P24 | Key | XXPQQXXXQQXXXXX | 312 | | | | |
| | More preferably | QQPQQPFPQQ | 313 | | | | |
| | Preferably | QQPFPQQQQ | 314 | | | | |
| | Key | QPFPQQ | 315 | | | | |
| P26 | Key | XXPQQXXXXQXXXXX | 316 | | | | |
| | Preferably | QPFPQQQ | 317 | | | | |
| | Key | QPFPQQ | 318 | | | | |
| P27 | Key | QQPQQPFPQQQQPXX | 319 | | | | |
| | Preferably | QPFPQQQQ | 320 | | | | |
| | Key | QPFPQQQ | 321 | | | | |
| P28 | Key | XXPQQXXPQQQXXXX | 322 | | | | |
| | Key | QPFPQQQQ | 323 | | | | |
| P32 | Key | XXXXQXXPQQQXXXX | 324 | | | | |
| | More preferably | QPFPQQQ | 325 | | | | |
| | Preferably | QPFPQQ | 326 | | | | |
| | Key | PFPQQ | 327 | | | | |
| P33 | Key | XXXXQXXPQQQXXXX | 328 | | | | |
| | More preferably | QPQQPFPQQQ | 329 | | | | |
| | Preferably | QPFPQQQQP | 330 | | | | |
| | Key | QPFPQQQ | 331 | | | | |
| P34 | Key | XXXQQXFXQQQXXXX | 332 | | | | |
| | Preferably | PQQPFPQQQQ | 333 | | | | |
| | Key | PQQPFPQ | 334 | | | | |
| | Key | FPQQQQPLI | 335 | | | | |
| P35 | Preferably | XXPQQXFPQXXXXXX | 336 | | | | |
| | Key | XXXQQXFPQXXXXXX | 337 | | | | |
| | Preferably | QPFPQQQ | 338 | | | | |
| | Key | QPFPQQ | 339 | | | | |
| P36 | Preferably | QQPFPQQQQP | 340 | | | | |
| | Key | QPFPQQQ | 341 | | | | |
| P50 | | | 342 | | | | |
| P52 | Preferably | QPQQPFP | 343 | | | | |
| | Key | QXXQPFX | 344 | | | | |
| | Key | QPFP | 345 | | | | |

**[Table 2-13]**

| E9 | | | | | | | |
|---|---|---|---|---|---|---|---|
| Spot No. 1,5,6 | | | | | | | |
| Protein name | Alpha/beta-gliadin MM1 P18573 | | SEQ ID NO | Food in which cross-reactivity was confirmed | Graph No. | Synthetic sequence | SEQ ID NO |
| 15-residue sequence | QVPLVQQQQFPGQQQ (positions 41-55 of SEQ ID NOs: 2, 10 and 12) | | 640 | | | | |
| P2 | Key | PLVQ | 641 | | | | |
| | Key | QQQFPGQQQ | 642 | | | | |
| P3 | More preferably | QVPLVQQ | 643 | | | | |
| | Preferably | QVPLVQ | 644 | | | | |
| | Key | QXXLXQQ | 645 | | | | |
| P7 | Key | QXXXVQQXXXXXXXX | 646 | | | | |
| | More preferably | QEQVPLVQQ | 647 | | | | |
| | Preferably | EQVPLVQ | 648 | | | | |
| | Key | EQXXXVQ | 649 | | | | |
| | Key | QVPLVQ | 650 | | | | |
| | Key | QQQFPGQQQ | 651 | | | | |
| P8 | Key | QXXLVQQXXXXXXXX | 652 | | | | |
| | More preferably | QVPLVQQ | 653 | | | | |
| | Preferably | PLVQQ | 654 | Wheat/rye | 2 | PLVQQ | 2886 |
| | Key | XLVQQ | 655 | Wheat/barley | 2 | KLVQQ | 2887 |
| | Preferably | QQQQFPGQQQ | 656 | | | | |
| | Key | QQFPGQQQ | 657 | Wheat/rye | 3 | QQFPGQQQ | 2888 |
| P12 | Key | QXXXVQQXXXXXXXX | 658 | 658 | | | |
| | Preferably | QVPLVQQ | 659 | | | | |
| | Key | QXXXVQQ | 660 | | | | |
| | Preferably | QVPLVQ | 661 | | | | |
| | Key | VPLVQ | 662 | | | | |
| | Key | QQFPGQQQ | 663 | | | | |
| P14 | More preferably | QQQQFPGQQQ | 664 | | | | |
| | Preferably | QQQFPGQQQ | 665 | | | | |
| | Key | QQFPGQQ | 666 | | | | |
| P17 | Preferably | FPGQQQP | 667 | | | | |
| | Key | FPGQQQ | 668 | | | | |
| P19 | Key | XXXXXQQQXXXXXQQ | 669 | Wheat/rye/ orchard grass | 2 | QVPLVQQQQFPGQQQ | 640 |
| | | | | | 2 | QQYYPQQQAFFLLQQ | 2885 |
| | More preferably | QEQVPLVQQQ | 670 | | | | |
| | Preferably | EQVPLVQQQ | 671 | | | | |
| | Key | EXXXXXQQQ | 672 | | | | |
| | Preferably | PLVQQQQFP | 673 | | | | |
| | Key | LVQQ | 674 | | | | |
| | Preferably | QQFPGQQQ | 675 | | | | |
| | Key | QFPGQQQ | 676 | | | | |
| P21 | Preferably | QVPLVOQ | 677 | | | | |
| | Key | QVPLVQ | 678 | | | | |
| | Key | QQFPGQQQ | 679 | | | | |
| P22 | Key | FPGQ | 680 | | | | |
| P24 | Preferably | QXPXVQQQQFPXQQQ | 681 | | | | |
| | Key | QXPXVQQXXXXXXQQ | 682 | | | | |
| | Preferably | QFPGQQQ | 683 | | | | |
| | Key | QFPXQQQ | 684 | | | | |
| P26 | Key | QVPLVQ | 685 | | | | |
| | Key | FPGQQ | 686 | | | | |
| P28 | Key | XXXXXQXQXXPXQXQ | 687 | | | | |
| | Key | PLVQQQQFPG | 688 | | | | |
| | Preferably | QQFPGQQQ | 689 | | | | |
| | Key | QXXPXQXQ | 690 | | | | |
| P32 | Key | XXPXXXXXQXXXQQQ | 691 | | | | |
| | Preferably | QQQQFPGQQQ | 692 | | | | |
| | Key | QQQFPGQQ | 693 | | | | |
| P33 | Key | VPLVQ | 694 | | | | |
| | Key | FPGQQ | 695 | | | | |
| P34 | Key | PLVQ | 696 | | | | |
| | Key | QQQFPGQQQ | 697 | | | | |
| P35 | More preferably | FPGQQQPFP | 698 | | | | |
| | Preferably | FPGQQQPF | 699 | | | | |
| | Key | FPGQQQ | 700 | | | | |
| P36 | Preferably | PLVQQQQF | 701 | | | | |
| | Key | PLVQQ | 702 | | | | |
| | Key | FPGQQQ | 703 | | | | |
| P50 | Key | PLVQQQQFP | 704 | | | | |
| | Key | QQQFPGQQ | 705 | | | | |

**[Table 2-15]**

| E11 | | | | | | | |
|---|---|---|---|---|---|---|---|
| Spot No. 1,5,6,14 | | | | | | | |
| Protein name | Alpha-gliadin (Fragment) A0A0E3Z522 | | SEQ ID NO | Food in which cross-reactivity was confirmed | Graph No. | Synthetic sequence | SEQ ID NO |
| 15-residue sequences | QPYPQPQPFPSQQPY (positions 61-75 of SEQ ID NOs: 2, 10, 12 and 28) | | 776 | | | | |
| P3 | Preferably | XXXXQPQPFPSQQXX | 777 | | | | |
| | Key | XXXXXXXPFPXQXXX | 778 | | | | |
| | Key | PQPQPFP | 779 | | | | |
| P4 | Preferably | QPYXXXXPXPSQXPY | 780 | | | | |
| | Key | XPYXXXXXXPXQXXY | 781 | | | | |
| | Preferably | PQPQPFPSQ | 782 | | | | |
| | Key | XXXXPXPSQ | 783 | | | | |
| | Preferably | PQPQPFP | 784 | | | | |
| | Key | QPFP | 785 | | | | |
| P5 | Preferably | XPYXQPXPFPSXXXX | 786 | | | | |
| | Key | XXXXQPXXXPSXXXX | 787 | | | | |
| | More preferably | QPQPFP | 788 | | | | |
| | Preferably | QPQPF | 789 | | | | |
| | Key | QPXPF | 790 | | | | |
| P7 | Preferably | XPYXQPXXFPSQXPY | 791 | | | | |
| | Key | XXYXQXXXXPSQXPY | 792 | | | | |
| | Further preferably | YPQPQPFPSQ | 793 | | | | |
| | More preferably | PQPQPFPSQ | 794 | | | | |
| | Preferably | XQPXXFPSQ | 795 | | | | |
| | Key | XQXXXXPSQ | 796 | | | | |
| | Most preferably | PQPFPSQQPY | 797 | | | | |
| | Further preferably | PFPSQQPYLQ | 798 | | | | |
| | More preferably | FPSQQPY | 799 | | | | |
| | Preferably | FPSQXPY | 800 | | | | |
| | Key | XPSQXPY | 801 | | | | |
| P8 | Preferably | QPYXXPQPFPSQQPY | 802 | | | | |
| | Key | XPYXXPQPFPSQQPY | 803 | | | | |
| | Preferably | PQPQPFP | 804 | | | | |
| | Key | XXPQPFP | 805 | Wheat/rye/barley | 3 | PQPQPFP | 2892 |
| P9 | Preferably | XPYXXXXXFPSQQXX | 806 | | | | |
| | Key | XXYXXXXXXPSQQXX | 807 | | | | |
| | Preferably | PQPQPFP | 808 | | | | |
| | Key | QPQPF | 809 | | | | |
| P10 | Key | XXYXXXQPXPXQQXX | 810 | | | | |
| | Key | PQPQPFP | 811 | | | | |
| P12 | Preferably | QPYPQPQPXPSQQPY | 812 | | | | |
| | Key | XXYXXXQPXPSQQXY | 813 | | | | |
| | Further preferably | PQPQPFPSQ | 814 | | | | |
| | More preferably | QPQPFPS | 815 | | | | |
| | Preferably | QPQPXPS | 816 | | | | |
| | Key | XXQPXPS | 817 | | | | |
| P14 | Key | XXYXQXXXXPSQQXY | 818 | | | | |
| | Key | QPFPSQ | 819 | | | | |
| P15 | Preferably | XPYXQPQPXPSQQXY | 820 | | | | |
| | Key | XXXXXXXPXPSQQXY | 821 | | | | |
| | Further preferably | YPQPQPFPSQ | 822 | | | | |
| | More preferably | PQPQPFPSQ | 823 | | | | |
| | Preferably | XQPQPXPSQ | 824 | | | | |
| | Key | XXXXPXPSQ | 825 | | | | |
| P19 | Key | XXYXXXXXXPSQQXX | 826 | | | | |
| | Key | YPQPQPFP | 827 | | | | |
| | Preferably | QPFPSQQPYL | 828 | | | | |
| | Key | XXXPSQQXXL | 829 | | | | |
| P21 | Preferably | QPYXQXQPXPSQQXX | 830 | | | | |
| | Key | XXYXXXQXXPSQQXX | 831 | | | | |
| | Preferably | PQPQPFP | 832 | | | | |
| | Key | QPQPF | 833 | | | | |
| P22 | Key | XPXXQPQXFPXQQXX | 834 | | | | |

**[Table 2-16]**

| | | | | | | | |
|---|---|---|---|---|---|---|---|
| P24 | Preferably | XPYXXPQPFPSQXXX | 835 | | | | |
| | Key | XXYXXXQPXPSXXXX | 836 | | | | |
| | Further preferably | PQPQPFPS | 837 | | | | |
| | More preferably | QPQPFPS | 838 | | | | |
| | Preferably | XPQPFPS | 839 | | | | |
| | Key | XXQPXPS | 840 | | | | |
| | Key | PQPF | 841 | | | | |
| P26 | Preferably | XXYXXXXPXPSQQPY | 842 | | | | |
| | Key | XXYXXXXXXPSQQXY | 843 | | | | |
| | Preferably | PQPQPFP | 844 | | | | |
| | Key | PQPQPF | 845 | | | | |
| P27 | Preferably | QPYXQPQPFPSQQPY | 846 | | | | |
| | Key | XPYXQPQPFPSQQPY | 847 | | | | |
| | Key | QPFPS | 848 | | | | |
| P28 | Preferably | QPYXXPQPFPXQQXY | 849 | | | | |
| | Key | QPYXXXXPXPXQQXX | 850 | | | | |
| | More preferably | PQPQPFP | 851 | | | | |
| | Preferably | QPQPFP | 852 | | | | |
| | Key | XPQPFP | 853 | | | | |
| P29 | Preferably | XPYXQPQPFPSXQPY | 854 | | | | |
| | Key | XXYXQPQPFPSXQPY | 855 | | | | |
| | More preferably | YPQPQPFPS | 856 | | | | |
| | Preferably | PQPQPFP | 857 | | | | |
| | Key | XQPQPFP | 858 | | | | |
| P30 | Preferably | XPYXQPQPFPSQQPY | 859 | | | | |
| | Key | XXYXXXXPFPXQQPY | 860 | | | | |
| | More preferably | YPQPQPFPS | 861 | | | | |
| | Preferably | PQPQPFP | 862 | | | | |
| | Key | XQPQPFP | 863 | | | | |
| P32 | Preferably | XXYXXPQPFPSQQXY | 864 | | | | |
| | Key | XXXXXXXXXPXQQXY | 865 | | | | |
| | More preferably | QPQPFPSQ | 866 | | | | |
| | Preferably | QPQPF | 867 | | | | |
| | Key | XPQPF | 868 | | | | |
| P33 | Key | XPYXXPQPXXSQQXX | 869 | | | | |
| | Key | PQPQPF | 870 | | | | |
| P34 | Preferably | QPXPQPQPFPXQQPY | 871 | | | | |
| | Key | QPXXXPQPFPXQQPY | 872 | | | | |
| | Preferably | PYPQPQPFP | 873 | | | | |
| | Key | PXXXPQPFP | 874 | | | | |
| | Key | PQPQP | 875 | | | | |
| P35 | Key | XXYXXPXPFPSQQXY | 876 | | | | |
| | Preferably | YPQPQPFPSQ | 877 | | | | |
| | Key | YXXPXPFPSQ | 878 | | | | |
| | Preferably | QPFPSQQP | 879 | | | | |
| | Key | XPFPSQQX | 880 | | | | |
| P50 | Key | XXYXXXQPFPSQQPX | 881 | | | | |
| | Key | QPQP | 882 | | | | |
| P52 | Preferably | QXYXQPXPFXXXXXX | 883 | | | | |
| | Key | XXXXQPXPFXXXXXX | 884 | | | | |
| | Key | PQPFPS | 885 | | | | |
| G6 | Preferably | XPYXXPQPFPSQXXX | 886 | | | | |
| | Key | XPYXXPQPFPXXXXX | 887 | | | | |
| | Preferably | PQPQPFP | 888 | | | | |
| | Key | XXPQPFP | 889 | | | | |
| G7 | Preferably | QPYPQPQPFPSXQPX | 890 | | | | |
| | Key | XPYPQPXPFPSXQXX | 891 | | | | |
| | Key | YPQP | 892 | | | | |
| G9 | Preferably | XXYXXPXXFXXQQPY | 893 | | | | |
| | Key | XXXXXXXXXXXQQPY | 894 | | | | |
| | Preferably | PQPQPFP | 895 | | | | |
| | Key | PQPQPF | 896 | | | | |
| | Preferably | PSQQPY | 897 | | | | |
| | Key | XXQQPY | 898 | | | | |

**[Table 2-26]**

| E19 | | | | | | | |
|---|---|---|---|---|---|---|---|
| Spot No. 7 | | | | | | | |
| Protein name | Gamma gliadin-A1 M9TG60 | | SEQ ID NO | Food in which cross-reactivity was confirmed | Graph No. | Synthetic sequence | SEQ ID NO |
| 15-residue sequences | SSLVSMILPRSDCKV (positions 211-225 of SEQ ID NO: 14) | | 1257 | | | | |
| P2 | Key | XXXXXXIXPXSDCKV | 1258 | | | | |
| | Key | LPRS | 1259 | | | | |
| P8 | Preferably | XXXVSMILPRSDCKV | 1260 | | | | |
| | Key | XXXXSMILPRSDXKV | 1261 | | | | |
| P9 | Preferably | XXXVSMILXRSDCKV | 1262 | | | | |
| | Key | XXXVSXILXXXXXKV | 1263 | | | | |
| P16 | More preferably | XXLVSMILPRSDCKV | 1264 | | | | |
| | Preferably | XXXXXMILPRSDCKV | 1265 | | | | |
| | Key | XXXXXXIXPRSDXKV | 1266 | | | | |
| P20 | Preferably | XXLVSMILPRSDCKV | 1267 | | | | |
| | Key | XXXXSMILPRSDCKV | 1268 | | | | |
| | Key | LPRS | 1269 | | | | |
| P21 | Preferably | XXXVSMILPRSDCKV | 1270 | | | | |
| | Key | XXXXSXILPRSXCKV | 1271 | | | | |
| P26 | More preferably | XXLVSMILPRSDCKV | 1272 | | | | |
| | Preferably | XXXVSMILPRSDCKV | 1273 | | | | |
| | Key | XXXXSMILPRSDCKV | 1274 | | | | |
| P29 | Preferably | XXXVSMILPRSDCKV | 1275 | | | | |
| | Key | XXXXSMILXRXDCKV | 1276 | | | | |
| | Key | LPRS | 1277 | | | | |
| P30 | Preferably | XXXVSMILPRSDCKV | 1278 | | | | |
| | Key | XXXXSMILPRSDCKV | 1279 | | | | |
| P33 | Preferably | XXXVSMILPRSDCKV | 1280 | | | | |
| | Key | XXXXSMILXRSDCKV | 1281 | | | | |
| | Key | LPRS | 1282 | | | | |
| P34 | Preferably | XXLVSXILXXSDCKV | 1283 | | | | |
| | Key | XXXVSXIXXXSDCKV | 1284 | | | | |
| P50 | Preferably | XXLXXMILXXSDXXX | 1285 | | | | |
| | Key | XXLXXMILXXXDXXX | 1286 | | | | |
| | Key | ILPRS | 1287 | | | | |
| P52 | Key | LPRS | 1288 | | | | |
| P53 | Preferably | XXXVXXILXRXDXKV | 1289 | | | | |
| | Key | XXXXXXILXRXDXXX | 1290 | | | | |
| | Key | LPRS | 1291 | | | | |
| G6 | More preferably | XXLVSMILXXSDCKV | 1292 | | | | |
| | Preferably | XXXVSMILXXSDCKV | 1293 | | | | |
| | Key | XXXVSXILXXXXXXX | 1294 | | | | |
| G7 | Key | VSMILPRSD | 1295 | | | | |
| | Key | ILPRSDCKV | 1296 | | | | |

**[Table 2-40]**

| E31 | | | | | | | |
|---|---|---|---|---|---|---|---|
| Spot No. 1,5,6 | | | | | | | |
| Protein name | Alpha-gliadin (Fragment) A0A0E3Z522 | | SEQ ID NO | Food in which cross-reactivity was confirmed | Graph No. | Synthetic sequence | SEQ ID NO |
| 15-residue sequences | QVPLVQQQQFPGQQQ (positions 41-55 of SEQ ID NOs: 2, 10 and 12) | | 1895 | | | | |
| P2 | Preferably | VPLVQQQ | 1896 | | | | |
| | Key | VPLVQQ | 1897 | | | | |
| | Key | QQFPGQQQ | 1898 | | | | |
| P3 | Key | QXXLVQQXXXXXXXX | 1899 | | | | |
| | Preferably | QVPLVQQ | 1900 | | | | |
| | Key | VPLVQ | 1901 | | | | |
| | Key | QQFPGQQ | 1902 | | | | |
| P4 | Key | QVXXXQQXXXXXXXX | 1903 | | | | |
| | Preferably | QVPLVQQ | 1904 | | | | |
| | Key | VPLVQ | 1905 | | | | |
| | Key | QFPGQQQ | 1906 | | | | |
| P7 | Key | QVXXXQQXXXXXXXX | 1907 | | | | |
| | Preferably | QVPLVQQ | 1908 | | | | |
| | Key | VPLVQ | 1909 | | | | |
| | Key | QFPGQQQ | 1910 | | | | |
| P8 | Key | QXXLVQQXXXXXXXX | 1911 | | | | |
| | Preferably | QVPLVQQQ | 1912 | | | | |
| | Key | QVPLVQQ | 1913 | Wheat/rye/barley | 3 | QVPLVQQ | 2912 |
| | Key | QQFPGQQ | 1914 | Wheat/rye | 3 | QQFPGQQ | 2913 |
| P9 | Key | QVPLVQQQQ | 1915 | | | | |
| | Key | QQQQFPGQQQ | 1916 | | | | |
| P10 | Key | QXXXVQQXXXXXXQQ | 1917 | | | | |
| | Preferably | QVPLVQQQQ | 1918 | | | | |
| | Key | VPLVQQQ | 1919 | | | | |
| | Key | LVQQQQFPGQ | 1920 | | | | |
| P12 | Preferably | QVPLVQQQ | 1921 | | | | |
| | Key | QVPLVQQ | 1922 | | | | |
| | Preferably | QQFPGQQQ | 1923 | | | | |
| | Key | FPGQQ | 1924 | | | | |
| P13 | Key | QFPGQQ | 1925 | | | | |
| P14 | Preferably | QQQFPGQQQ | 1926 | | | | |
| | Key | QFPGQQ | 1927 | | | | |
| P15 | Key | VPLVQQQ | 1928 | | | | |
| | Preferably | QFPGQQQ | 1929 | | | | |
| | Key | FPGQQQ | 1930 | | | | |
| P17 | Key | QXXXVQQXXXXXXXQ | 1931 | | | | |
| | Preferably | QVPLVQQQ | 1932 | | | | |
| | Key | VPLVQQ | 1933 | | | | |
| P19 | Preferably | XXXXVQQQQXXXQQQ | 1934 | | | | |
| | Key | XXXXXQQXXXXXXQQ | 1935 | | | | |
| | Key | QVPLVQQ | 1936 | | | | |
| | Preferably | QQFPGQQQ | 1937 | | | | |
| | Key | QFPGQQ | 1938 | | | | |
| P21 | Preferably | QVPLVQQ | 1939 | | | | |
| | Key | VPLVQ | 1940 | | | | |
| | More preferably | QQFPGQQQ | 1941 | | | | |
| | Preferably | QQFPGQQ | 1942 | | | | |
| | Key | QFPGQQ | 1943 | | | | |
| P22 | More preferably | QFPGQQQ | 1944 | | | | |
| | Preferably | FPGQQ | 1945 | | | | |
| | Key | FPGQ | 1946 | | | | |
| P24 | Key | XXXXXQQXXXXXQQQ | 1947 | | | | |
| | Preferably | QVPLVQQQ | 1948 | | | | |
| | Key | VPLVQQ | 1949 | | | | |
| | More preferably | QQQFPGQQQ | 1950 | | | | |
| | Preferably | QFPGQQQ | 1951 | | | | |
| | Key | QFPGQQ | 1952 | | | | |

**[Table 2-41]**

| | | | | | | | |
|---|---|---|---|---|---|---|---|
| P26 | Key | XXXXXXXQXXPGQQX | 1953 | | | | |
| | Preferably | QQFPGQQ | 1954 | | | | |
| | Key | QFPGQ | 1955 | | | | |
| P28 | Preferably | XXXXXXXQXXPGQQQ | 1956 | | | | |
| | Key | XXXXXXXXXXPGQQQ | 1957 | | | | |
| | More preferably | QQFPGQQQ | 1958 | | | | |
| | Preferably | QFPGQQ | 1959 | | | | |
| | Key | XXPGQQ | 1960 | | | | |
| P32 | Preferably | QQQQFPGQQ | 1961 | | | | |
| | Key | QQQFPGQQ | 1962 | | | | |
| P33 | Key | XXXXXQXQQXXXQQQ | 1963 | | | | |
| | More preferably | QQQFPGQQ | 1964 | | | | |
| | Preferably | QQFPGQQ | 1965 | | | | |
| | Key | QFPGQQ | 1966 | | | | |
| P34 | Key | VPLVQQQ | 1967 | | | | |
| | Key | QFPGQQ | 1968 | | | | |
| P35 | More preferably | QVXLVQQXXXPGQQQ | 1969 | | | | |
| | Preferably | QXXLVQQXXXXXXXQ | 1970 | | | | |
| | Key | XXXLVQQXXXXXXXX | 1971 | | | | |
| | More preferably | QVPLVQQQQ | 1972 | | | | |
| | Preferably | QVPLVQQQ | 1973 | | | | |
| | Key | VPLVQQ | 1974 | | | | |
| P50 | Key | LVQQQQFP | 1975 | | | | |
| G6 | Key | XXXXXXXXXFPGQXX | 1976 | | | | |

**[Table 2-47]**

| E36 Spot No. 1,6,14 | | | | | | | |
|---|---|---|---|---|---|---|---|
| Protein name | Alpha/beta-gliadin MM1 P18573 | | SEQ ID NO | Food in which cross-reactivity was confirmed | Graph No. | Synthetic sequence | SEQ ID NO |
| 15-residue sequences | YSQPQQPISQQQQQQ (positions 121-135 of SEQ ID NO: 2, positions 107-121 of SEQ ID NOs: 12 and 28) | | 2154 | | | | |
| P2 | More preferably | YSQPQQPISQ | 2155 | | | | |
| | Preferably | YSQPQQPIS | 2156 | | | | |
| | Key | SQPQQ | 2157 | | | | |
| P12 | Key | YSQPQQPISQ | 2158 | | | | |
| P20 | Preferably | YSQPQQPI | 2159 | | | | |
| | Key | SQPQQ | 2160 | | | | |
| P2 1 P21 | Preferably | PQQPISQQQ | 2161 | | | | |
| | Key | QQPISQ | 2162 | | | | |
| P22 | Preferably | SQPQQPISQ | 2163 | | | | |
| | Key | SQPQQ | 2164 | | | | |
| P24 | More preferably | YSQPQQPISQ | 2165 | | | | |
| | Preferably | SQPQQPIS | 2166 | | | | |
| | Key | SQPQQ | 2167 | | | | |
| P25 | Key | YXXXXXXXXXXQQQX | 2168 | | | | |
| | More preferably | YSQPQQPI | 2169 | | | | |
| | Preferably | SQPQQP | 2170 | | | | |
| | Key | SQPQQ | 2171 | | | | |
| | Key | QQPISQQQQQ | 2172 | | | | |
| P26 | More preferably | YSQPQQPISQ | 2173 | | | | |
| | Preferably | YSQPQQ | 2174 | | | | |
| | Key | SQPQQ | 2175 | | | | |
| P27 | Preferably | YSQPQQPISQ | 2176 | | | | |
| | Key | SQPQQ | 2177 | | | | |
| P28 | More preferably | YSQPQQPISQ | 2178 | | | | |
| | Preferably | YSQPQQPI | 2179 | | | | |
| | Key | SQPQQ | 2180 | | | | |
| P29 | More preferably | YSQPQQPISQ | 2181 | | | | |
| | Preferably | SQPQQ | 2182 | | | | |
| | Key | QPQQ | 2183 | | | | |
| P30 | More preferably | YSQPQQPISQ | 2184 | | | | |
| | Preferably | YSQPQQPI | 2185 | | | | |
| | Key | SQPQQ | 2186 | | | | |
| P31 | Key | SQPQQ | 2187 | | | | |
| P32 | Key | YXQXXXXXXXXQQXX | 2188 | | | | |
| | Preferably | YSQPQQPISQ | 2189 | | | | |
| | Key | QPQQ | 2190 | | | | |
| P33 | Preferably | SQPQQ | 2191 | | | | |
| | Key | QPQQ | 2192 | | | | |
| P34 | Key | SQPQQ | 2193 | | | | |
| P35 | Preferably | YSQPQQ | 2194 | | | | |
| | Key | SQPQQ | 2195 | | | | |
| P36 | Preferably | YSQPQQPISQ | 2196 | | | | |
| | Key | SQPQQ | 2197 | | | | |
| P50 | Key | SQPQQ | 2198 | | | | |
| P53 | Key | YXXXQXXXXXQQQXX | 2199 | | | | |
| | More preferably | YSQPQQPISQ | 2200 | | | | |
| | Preferably | YSQPQQPI | 2201 | | | | |
| | Key | SQPQQ | 2202 | | | | |
| | Key | SQQQQQQ | 2203 | | | | |
| P54 | Key | YXXXXXXXXXQQQQQ | 2204 | | | | |
| | Preferably | YSQPQQPIS | 2205 | | | | |
| | Key | SQPQQPI | 2206 | | | | |
| | Preferably | QQPISQQQQQ | 2207 | | | | |
| | Key | XXXXXXQQQQ | 2208 | | | | |
| G5 | Preferably | YSQPQQ | 2209 | | | | |
| | Key | SQPQQ | 2210 | | | | |
| G6 | Preferably | YSQPQQPISQ | 2211 | | | | |
| | Key | SQPQQ | 2212 | | | | |
| G7 | Preferably | YSQPQQPISQ | 2213 | | | | |
| | Key | QPQQ | 2214 | | | | |
| G9 | Preferably | YSOPOOPIS | 2215 | | | | |
| | Key | SQPQQ | 2216 | | | | |

**[Table 2-48]**

| E37 Spot No. 11, 12 | | | | | | | |
|---|---|---|---|---|---|---|---|
| Protein name | Globulin 3 B7U6L4 | | SEQ ID NO | Food in which cross-reactivity was confirmed | Graph No. | Synthetic sequence | SEQ ID NO |
| 15-residue sequences | VSRLLRGIRNYRVAI (positions 161-175 of SEQ ID NO: 22) | | 2217 | | | | |
| P2 | Key | SRLLRGIRNY | 2218 | | | | |
| | Preferably | IRNYRVAI | 2219 | | | | |
| | Key | NYRV | 2220 | | | | |
| P7 | Key | IRNYR | 2221 | | | | |
| P8 | Key | RLLRGIRNY | 2222 | Wheat | 3 | RLLRGIRNY | 2917 |
| | Key | IRNYRVA | 2223 | Wheat | 3 | IRNYRVA | 2918 |
| P16 | Key | IRNYRVA | 2224 | | | | |
| P17 | Key | VSRLLRGIRN | 2225 | | | | |
| | Key | RNYR | 2226 | | | | |
| P18 | Key | LLRGIRN | 2227 | | | | |
| | Key | NYRVAI | 2228 | | | | |
| P21 | Key | XXXXXRGIXNYXXXX | 2229 | | | | |
| | Key | IRNYRV | 2230 | | | | |
| P28 | Key | IRNY | 2231 | | | | |
| P29 | Key | XXXXXRGXXNYXXXI | 2232 | | | | |
| | Preferably | IRNYRVAI | 2233 | | | | |
| | Key | RNYR | 2234 | | | | |
| P30 | Key | SRLLRGIRN | 2235 | | | | |
| | Preferably | IRNYRVAI | 2236 | | | | |
| | Key | NYRV | 2237 | | | | |
| P31 | Key | LLRGIRNYRV | 2238 | | | | |
| | Key | NYRVAI | 2239 | | | | |
| P32 | Key | IRNYRVAI | 2240 | | | | |
| P34 | Key | RLLRGIRN | 2241 | | | | |
| P35 | Key | IRNYRVAI | 2242 | | | | |
| P36 | Key | RLLRGIRNY | 2243 | | | | |
| | Key | RNYRVA | 2244 | | | | |
| P50 | Preferably | SRLLRGIR | 2245 | | | | |
| | Key | SRLLR | 2246 | | | | |
| | Key | LLRGIRNYRV | 2247 | | | | |
| | Key | IRNYRVAI | 2248 | | | | |
| P52 | Key | XXXXLXXIXNYXXXX | 2249 | | | | |
| | Preferably | SRLLRGIRN | 2250 | | | | |
| | Key | SRLLRGIR | 2251 | | | | |
| | More preferably | LLRGIRNYRV | 2252 | | | | |
| | Preferably | RNYRVAI | 2253 | | | | |
| | Key | NYRV | 2254 | | | | |
| P53 | Preferably | LLRGIRNYRV | 2255 | | | | |
| | Key | LRGIRN | 2256 | | | | |
| P55 | Key | SRLLRGIRN | 2257 | | | | |
| | Preferably | LRGIRNYRVA | 2258 | | | | |
| | Key | RNYRV | 2259 | | | | |
| G1 | Key | SRLLR | 2260 | Wheat/barley | 4 | SRLLR | 2919 |
| | Key | LLRGIRNYRV | 2261 | Wheat | 4 | LLRGIRNYRV | 2920 |
| | Key | RNYRVAI | 2262 | Wheat | 4 | RNYRVAI | 2921 |
| G6 | Key | SRLLR | 2263 | | | | |
| | Key | NYRVAI | 2264 | | | | |

**[Table 2-55]**

| E43 Spot No. I | | | | | | | |
|---|---|---|---|---|---|---|---|
| Protein name | Alpha/beta-gliadin MM1 P18573 | | SEQ ID NO | Food in which cross-reactivity was confirmed | Graph No. | Synthetic sequence | SEQ ID NO |
| 15-residue sequences | LQQSTYQLVQQLCCQ (positions 181-195 of SEQ ID NO: 2) | | 2545 | | | | |
| P2 | More preferably | LQQSTYQLVQ | 2546 | | | | |
| | Preferably | QSTYQLVQQL | 2547 | | | | |
| | Key | QSTY | 2548 | | | | |
| | Key | OLVOOLCC | 2549 | | | | |
| P7 | Key | LXQSTYXLVXXXXXX | 2550 | | | | |
| | More preferably | YQLVQQLCC | 2551 | | | | |
| | Preferably | QLVQQLCC | 2552 | | | | |
| | Key | QLVQ | 2553 | | | | |
| P20 | Key | LOOSTYOLVO | 2554 | | | | |
| | Key | OSTYOLVOOL | 2555 | | | | |
| | Key | OLVOOLCC | 2556 | | | | |
| P21 | Key | XXXXTYXLXXXLXXX | 2557 | | | | |
| | Key | QSTYQLVQQL | 2558 | | | | |
| | Key | LVQQLCC | 2559 | | | | |
| P22 | Key | LQQSTYQLVQ | 2560 | | | | |
| | Key | LVQQL | 2561 | | | | |
| P24 | More preferably | QSTYQLVQQL | 2562 | | | | |
| | Preferably | QLVQQLCC | 2563 | | | | |
| | Key | OLVOO | 2564 | | | | |
| P25 | Key | QSTYQLVQ | 2565 | | | | |
| | Key | YQLVQQLCC | 2566 | | | | |
| P26 | Key | QLVQQ | 2567 | | | | |
| P27 | Key | XXXSTYQLVXXLXCX | 2568 | | | | |
| | Preferably | QLVQQL | 2569 | | | | |
| | Key | QLVQQ | 2570 | | | | |
| P28 | Preferably | XQQSTYQLVXXLXCX | 2571 | | | | |
| | Key | XXXSXYQLVXXXXXX | 2572 | | | | |
| | More preferably | YQLVQQL | 2573 | | | | |
| | Preferably | QLVQQL | 2574 | | | | |
| | Key | QLVQ | 2575 | | | | |
| P29 | Preferably | XXXSTYQLXXXXXCX | 2576 | | | | |
| | Key | XXXSTYQLXXXXXXX | 2577 | | | | |
| | Key | YQLVQQL | 2578 | | | | |
| P30 | Key | YQLVQQ | 2579 | | | | |
| P31 | Preferably | XQXSTYQLVXXXXCX | 2580 | | | | |
| | Key | XXXXTYXLVXXXXXX | 2581 | | | | |
| | More preferably | YQLVQQL | 2582 | | | | |
| | Preferably | QLVQQ | 2583 | | | | |
| | Key | QLVQ | 2584 | | | | |
| P32 | Preferably | LQQSTYQLVQ | 2585 | | | | |
| | Key | LQQSTYQL | 2586 | | | | |
| | Preferably | QLVQQLCC | 2587 | | | | |
| | Key | QQLC | 2588 | | | | |
| P34 | More preferably | QSTYQLVQQL | 2589 | | | | |
| | Preferably | QLVQQLCC | 2590 | | | | |
| | Key | QLVQQL | 2591 | | | | |
| P35 | Preferably | YQLVQQ | 2592 | | | | |
| | Key | QLVQ | 2593 | | | | |
| P36 | Preferably | QLVQQL | 2594 | | | | |
| | Key | VQQL | 2595 | | | | |
| P53 | Key | XXXSXYXXXXXLXCX | 2596 | | | | |
| | Key | QQSTYQLV | 2597 | | | | |
| | More preferably | YQLVQQLCC | 2598 | | | | |
| | Preferably | QLVQQLCC | 2599 | | | | |
| | Key | QQLC | 2600 | | | | |
| P54 | Preferably | QQSTYQLVQ | 2601 | | | | |
| | Key | OOSTYOLV | 2602 | | | | |
| | Key | OLVOOLC | 2603 | | | | |
| G3 | Preferably | YQLVQ | 2604 | | | | |
| | Key | QLVQ | 2605 | | | | |
| G5 | Key | OLVOOL | 2606 | | | | |
| G6 | Key | LOOSTYOLVO | 2607 | | | | |
| | Key | OLVOOL | 2608 | | | | |
| G7 | Key | OLVOOLCC | 2609 | | | | |
| G9 | Key | LOOSTYOLV | 2610 | | | | |
| | Key | OSTYOLVOOL | 2611 | | | | |
| | Key | OLVOOLCC | 2612 | | | | |

**[Table 2-56]**

| E44 Spot No. 11,12 | | | | | | | |
|---|---|---|---|---|---|---|---|
| Protein name | Globulin 3 B7U6L4 | | SEQ ID NO | Food in which cross-reactivity was confirmed | Graph No. | Synthetic sequence | SEQ ID NO |
| 15-residue sequences | SAKPLLASLSKRVLT (positions 261-275 of SEQ ID NO: 22) | | 2613 | | | | |
| P2 | Key | AKPLL | 2614 | | | | |
| | Key | SLSKRV | 2615 | | | | |
| P24 | Key | AKPLL | 2616 | | | | |
| | Key | SKRV | 2617 | | | | |
| P32 | Key | XXXXXLASLSKXXLX | 2618 | | | | |
| | Key | LASLSK | 2619 | | | | |
| G2 | Key | XXXXXXXXLSXRXLX | 2620 | | | | |
| | Key | LLAS | 2621 | | | | |
| G3 | Key | SXXXXLAXXXXXVLX | 2622 | | | | |
| | Preferably | SAKPLL | 2623 | | | | |
| | Key | AKPLL | 2624 | | | | |
| | Preferably | ASLSKRV | 2625 | | | | |
| | Key | LSKRV | 2626 | | | | |
| G7 | Key | LLASLSKRV | 2627 | | | | |
| G9 | Preferably | XXXXLLASLSKXXXX | 2628 | | | | |
| | Key | XXXXLLAXLSKXXXX | 2629 | | | | |
| | Key | LASLSK | 2630 | | | | |

In Table 2, WDEIA cases in wheat-allergic patients are represented by P2 to P36, children of cases with general immediate type allergy to wheat, which requires no exercise for its development, are represented by P50 to P57, and adults are represented by G1 to G9.

The present invention provides polypeptides comprising the amino acid sequences defined in (E4), (E9), (E11), (E19), (E31), (E36), (E37), (E43) and (E44), which comprise or consist of the amino acid sequences of SEQ ID NOs: 29-2932 (except for SEQ ID NOs: 138, 227, 259, 269, 273, 342, 399 and 2894) described in Table 2 mentioned later as polypeptides comprising an amino acid sequence specifically binding to an IgE antibody from an allergic patient. The polypeptides (E4), (E9), (E11), (E19), (E31), (E36), (E37), (E43) and (E44) each have an amino acid sequence (hereinafter also referred to as an "epitope") binding to an IgE antibody derived from a protein described in "Origin" of Table 2.
(1) Alpha/beta-gliadin MM1 protein-derived: (E9), (E31), (E11), (E36) and (E43);
(7) Gamma-gliadin A1 protein-derived: (E4) and (E19); and
(11) Globulin 3 protein-derived: (E37) and (E44).

The amino acid sequences defined in (E9) and (E31) are the same sequences.

The epitope antigen of the present invention is not limited and preferably comprises at least one of the following polypeptides:
(E4) a polypeptide comprising at least one amino acid sequence selected from the amino acid sequences of SEQ ID NOs: 281-341 and 343-345;
(E9) a polypeptide comprising at least one amino acid sequence selected from the amino acid sequences of SEQ ID NOs: 640-705 and 2885-2888;
(E11) a polypeptide comprising at least one amino acid sequence selected from the amino acid sequences of SEQ ID NOs: 776-898 and 2892;
(E19) a polypeptide comprising at least one amino acid sequence selected from the amino acid sequences of SEQ ID NOs: 1257-1296;
(E31) a polypeptide comprising at least one amino acid sequence selected from the amino acid sequences of SEQ ID NOs: 1895-1976, 2912 and 2913;
(E36) a polypeptide comprising at least one amino acid sequence selected from the amino acid sequences of SEQ ID NOs: 2154-2216;
(E37) a polypeptide comprising at least one amino acid sequence selected from the amino acid sequences of SEQ ID NOs: 2217-2264 and 2917-2921;
(E43) a polypeptide comprising at least one amino acid sequence selected from the amino acid sequences of SEQ ID NOs: 2545-2612; and
(E44) a polypeptide comprising at least one amino acid sequence selected from the amino acid sequences of SEQ ID NOs: 2613-2630;

As described in Table 2, the polypeptides (E4), (E9), (E11), (E19), (E31), (E36), (E37), (E43) and (E44) mentioned above as a preferred embodiment have a specific sequence identified as an epitope binding to an IgE antibody in Examples herein. The epitope antigen of the present invention may include variants described below, etc., in addition to the aforementioned polypeptides (E4), (E9), (E11), (E19), (E31), (E36), (E37), (E43) and (E44) according to a preferred embodiment. Hereinafter, the forms (variants) that may be included in the epitope antigen of the present invention will be described.

9 types of sequences, i.e., SEQ ID NOs: 281, 640, 776, 1257, 1895, 2154, 2217, 2545 and 2613, described in "Common 15-residue sequence" of Table 2 are common sequences of 15 amino acid residues identified as epitopes binding to IgE antibodies for the epitopes of the polypeptides (E4), (E9), (E11), (E19), (E31), (E36), (E37), (E43) and (E44) by epitope mapping based on overlapping. In one embodiment, the present invention provides polypeptides comprising these amino acid sequences or consisting of these amino acid sequences. The epitope sequence contained in the polypeptide of the present invention can be all the 15 amino acid residues of this common epitope, or a portion thereof. The epitope sequence is of at least 4 amino acid residues, at least 5 amino acid residues, at least 6 amino acid residues, at least 7 amino acid residues, at least 8 amino acid residues, at least 9 amino acid residues, at least 10 amino acid residues, at least 11 amino acid residues, at least 12 amino acid residues, at least 13 amino acid residues or at least 14 amino acid residues.

In Examples herein, polypeptides consisting of 4 amino acid residues (e.g., SEQ ID NOs: 30, 58, 61, 222, 238 and 345) were confirmed as epitopes binding to IgE antibodies in many samples. Moreover, the binding activity of polypeptides of 5 or more amino acid residues against IgE antibodies was also confirmed in many samples. Accordingly, the presence of at least 4 amino acid residues is useful as an epitope sequence.

In one embodiment, variants of the polypeptide of the present invention include polypeptides comprising at least 4 amino acid residues of each of the specific amino acid sequences defined in (E4), (E9), (E11), (E19), (E31), (E36), (E37), (E43) and (E44). For example, variants of the polypeptide comprising the amino acid sequence (E4) may include "polypeptides comprising at least 4 amino acid residues in at least one of the amino acid sequences of SEQ ID NOs: 281-341 and 343-345". Preferably, such variants comprise at least 4 amino acid residues, at least 5 amino acid residues, at least 6 amino acid residues, at least 7 amino acid residues, at least 8 amino acid residues, at least 9 amino acid residues, at least 10 amino acid residues, at least 11 amino acid residues, at least 12 amino acid residues, at least 13 amino acid residues, or at least 14 amino acid residues in at least one of the amino acid sequences of SEQ ID NOs: 29-130 and 2880. The same holds true for the amino acid sequences defined in (E9), (E11), (E19), (E31), (E36), (E37), (E43) and (E44).

In one embodiment, variants of each polypeptide of the present invention are not limited and comprise amino acid residues described below.
E9: at least 4 amino acid residues of VPLVQ;
E19: at least 4 amino acid residues of SMILPRSDCKV;
E31: at least 4 amino acid residues of QVPLV;
E36: at least 4 amino acid residues of YSQPQQ, at least 4 amino acid residues of PISQ, and/or at least 4 amino acid residues of SQQQ;
E37: at least 4 amino acid residues of SRLLR, and/or at least 4 amino acid residues of IRNYRYV,
E43: at least 4 amino acid residues of QLVQQ, and/or at least 4 amino acid residues of QQLCC; and
E44: at least 4 amino acid residues of LASL, and/or at least 4 amino acid residues of SKRV;

As shown in Table 2, the aforementioned sequences were found as sequences common in a plurality of sequences as to the specific sequences of each epitope binding to IgE antibodies in Examples.

In Table 2, the "preferred" sequence is a shorter partial sequence capable of functioning as an epitope in "Common 15-residue sequence". The "more preferred" sequence is a sequence more preferable than the aforementioned shorter partial sequence for improving binding activity against IgE antibodies. The "key" sequence represents a sequence deemed to be particularly important in "Common 15-residue sequence" or "key" sequence. The amino acid sequence of "X" in the sequence of "key" is an amino acid residue confirmed to have binding activity against IgE antibodies that remains even after exchange to any given alanine (glycine when the original amino acid residue is alanine) in alanine/glycine scanning. Accordingly, X is any given amino acid residue, preferably alanine (or glycine). The sequence of "key" comprising no sequence of "X" was not found to have the amino acid residue confirmed to have binding activity against IgE antibodies that remains in alanine/glycine scanning.

As for the epitopes described in Table 2, the amino acid residue of X is an amino acid residue confirmed to have binding activity against IgE antibodies that remains even after change. In the present invention, preferably, one or more amino acid residues of X in the "key sequence" corresponding to each "preferred sequence" may be substituted by any given amino acid residue. In one embodiment, for example, in SEQ ID NO: 281 which is a preferred sequence, the corresponding key sequence is a plurality of sequences including SEQ ID NOs: 284 and 288. Hereinafter, the same holds true for other "preferred sequences" and "key sequences" of the polypeptide (E4), and the polypeptides (E9), (E11), (E19), (E31), (E36), (E37), (E43) and (E44).

The number of amino acid residues that may be substituted is not limited and is preferably no more than 6, no more than 5, no more than 4, no more than 3, no more than 2 or no more than 1. Hereinafter, the same holds true for the polypeptides (E9), (E11), (E19), (E31), (E36), (E37), (E43) and (E44).

Each sequence described in the right column "Synthetic sequence" and/or "SEQ ID NO" of graph No. of Table 2 is a sequence confirmed to have epitope cross-reaction in Example 5. "Confirmed to have epitope cross-reactivity" means that an IgE antibody in the serum of each allergic patient exhibits binding activity higher than (specifically, larger than "1" in Figs. 7 to 12) that of an IgE antibody in the serum of a nonallergic subject. Accordingly, in one embodiment, the polypeptide of the present invention includes polypeptides comprising these amino acid sequences or consisting of these amino acid sequences. In one embodiment, the IgE antibody in the serum of each allergic patient exhibits at least 1.05 times, at least 1.10 times, at least 1.15 times, at least 1.20 times or at least 1.25 times higher than the binding activity of an IgE antibody in the serum of a nonallergic subject against the polypeptide of the present invention.

As referred to herein, in a preferred embodiment, the "polypeptides comprising the amino acid sequences defined in (E4), (E9), (E11), (E19), (E31), (E36), (E37), (E43) and (E44)" include a polypeptide comprising each amino acid sequence of the aforementioned polypeptides (E4), (E9), (E11), (E19), (E31), (E36), (E37), (E43) and (E44) or consisting of each amino acid sequence thereof, and any form (variant) in which amino acid residues are substituted as mentioned above. "Comprising each amino acid sequence of SEQ ID NO: xx" means that the amino acid sequence may additionally comprise any given amino acid sequence without influencing the binding between each amino acid sequence of SEQ ID NO: (including their aforementioned substituted forms) and an IgE antibody (i.e., their functions as an epitope).

Amino acid residues other than the specifically defined amino acid sequences in the polypeptides may be arbitrarily selected without influencing binding to IgE antibodies (i.e., their functions as epitopes). It is desirable to appropriately select these amino acid residues, preferably, from the sequences of their respective original epitopes or the sequences of their respective original proteins, though the amino acid residues are not limited thereto. For example, SEQ ID NO: 294 is defined only by "QPQQP", and if other amino acid residues are added thereto, it is desirable to appropriately select the amino acid residues from the original sequence of SEQ ID NO: 281. Further, for the sequences described as (E4) in Table 2, it is desirable to add amino acid residues of a sequence derived from the original protein as defined in (7) (corresponding to spot (7)).

The polypeptides comprising the amino acid sequences defined above in (E4), (E9), (E11), (E19), (E31), (E36), (E37), (E43) and (E44) may be prepared by a technique of chemical synthesis such as solid-phase peptide synthesis. Alternatively, polypeptides comprising an epitope may be obtained by expressing them as recombinant polypeptides using a genetic recombination technique well known to those skilled in the art and by separating and purifying them using protein purification methods well known to those skilled in the art. Two or more of the polypeptides may be joined together in combination, or repeats of one epitope may be joined together. In this case, the binding activity against Ig antibodies is generally improved.

The lengths of the polypeptides comprising the amino acid sequences defined above in (E4), (E9), (E11), (E19), (E31), (E36), (E37), (E43) and (E44) are not particularly limited. In a preferred embodiment, the lengths of the polypeptides comprising the amino acid sequences defined above in (E4), (E9), (E11), (E19), (E31), (E36), (E37), (E43) and (E44) can be no more than 500 amino acids, no more than 300 amino acids, no more than 200 amino acids, no more than 100 amino acids, no more than 50 amino acids, no more than 30 amino acids, no more than 20 amino acids, no more than 15 amino acids, no more than 10 amino acids, or no more than 5 amino acids. In the case of a polypeptide in which repeats of one or more of the amino acid sequences defined above in (E4), (E9), (E11), (E19), (E31), (E36), (E37), (E43) and (E44) are joined together, the length of such an amino acid sequence moiety in a preferred embodiment may be no more than 1000 amino acids, no more than 750 amino acids, no more than 500 amino acids, no more than 250 amino acids, no more than 100 amino acids, no more than 75 amino acids, no more than 50 amino acids, no more than 30 amino acids, no more than 15 amino acids, no more than 10 amino acids or no more than 5 amino acids. The number of amino acid residues described in the preferred embodiments as the lengths of the aforementioned polypeptides is the total length of sequences flanking a spacer (excluding the spacer).

Preferably, the antigen of the present invention specifically binds to an IgE antibody from an allergic patient.

### Diagnosis kit and method (2)

The present invention provides a method for providing an indicator for diagnosing an allergy in a subject, the method comprising the steps of:
(i) contacting a sample obtained from the subject with an antigen, wherein the sample is a solution comprising an IgE antibody;
(ii) detecting binding between the IgE antibody present in the sample obtained from the subject and the antigen; and
(iii) when the binding between the IgE antibody in the subject and the antigen is detected, an indicator of the fact that the subject is allergic is provided;
wherein the antigen is a polypeptide which is at least one of the polypeptides comprising the amino acid sequences defined above in (E4), (E9), (E11), (E19), (E31), (E36), (E37), (E43) and (E44), or a polypeptide in which two or more of the polypeptides comprising the amino acid sequences defined above in (E4), (E9), (E11), (E19), (E31), (E36), (E37), (E43) and (E44) are joined together via or without a spacer.

Hereinafter, the polypeptide which is at least one of the polypeptides comprising the amino acid sequences defined above in (E4), (E9), (E11), (E19), (E31), (E36), (E37), (E43) and (E44), or the polypeptide in which two or more of the polypeptides comprising the amino acid sequences defined above in (E4), (E9), (E11), (E19), (E31), (E36), (E37), (E43) and (E44) are joined together via or without a spacer is also referred to as the "antigen including (E4), (E9), (E11), (E19), (E31), (E36), (E37), (E43) and (E44)" in the present specification. The type of the spacer is not particularly limited, and an ordinary spacer that is used by those skilled in the art for joining together two or more peptides can be used. The spacer may be, for example, a hydrocarbon chain such as Acp(6)-OH, or a polypeptide such as an amino acid chain.

In the case of joining together the polypeptides comprising the amino acid sequences defined above in (E4), (E9), (E11), (E19), (E31), (E36), (E37), (E43) and (E44) via or without a spacer, the number of polypeptides to be joined together is not particularly limited. In one embodiment, the number of polypeptides to be joined together is at least 2, at least 3, at least 4, at least 5, at least 6, at least 8, at least 10 or at least 15. In one embodiment, the number of polypeptides to be joined together is no more than 30, no more than 20, no more than 15, no more than 10, no more than 8, no more than 6, no more than 5, no more than 3 or no more than 2.

The same or different repeats of the polypeptides comprising the amino acid sequences defined above in (E4), (E9), (E11), (E19), (E31), (E36), (E37), (E43) and (E44) may be joined together. In such a case of joining together two or more of the polypeptides comprising the amino acid sequences defined above in (E4), (E9), (E11), (E19), (E31), (E36), (E37), (E43) and (E44), the polypeptide of the present invention can also be applied to the method, the kit or the composition of the present invention.

The sample obtained from a subject is as described above in the subsection titled "Diagnosis kit and method (1)".

Detection of contact and binding between the sample obtained from a subject and the polypeptide can be carried out by using a known method described above in the subsection titled "Diagnosis kit and method (1)", such as ELISA (Enzyme-Linked Immunosorbent Assay), sandwich immunoassay, immunoblotting, immunoprecipitation, or immunochromatography.

The polypeptides comprising the amino acid sequences defined above in (E4), (E9), (E11), (E19), (E31), (E36), (E37), (E43) and (E44) may be provided in a state immobilized on a carrier. In this case, the steps (i) and (ii) mentioned above can be carried out using ELISA, sandwich immunoassay, immunochromatography, surface plasmon resonance, or the like. The step (i) mentioned above can be carried out by contacting the sample obtained from a subject with a surface on which the polypeptides comprising the amino acid sequences defined above in (E4), (E9), (E11), (E19), (E31), (E36), (E37), (E43) and (E44) are immobilized. The IgE antibody from the subject may be used in a state immobilized on a carrier, and binding to the polypeptides comprising the amino acid sequences defined above in (E4), (E9), (E11), (E19), (E31), (E36), (E37), (E43) and (E44) may be detected by the aforementioned technique. In order to establish binding to a carrier or a space from the carrier, or to facilitate contact of a polypeptide with an antibody, a spacer or a tag such as biotin may be added to the N terminus or C terminus of the polypeptide. In the case of binding to biotin, the carrier preferably has avidin.

The polypeptides comprising the amino acid sequences defined above in (E4), (E9), (E11), (E19), (E31), (E36), (E37), (E43) and (E44) may be in a state unimmobilized on a carrier. In this case, flow cytometry or the like can be used in the aforementioned steps (i) and (ii), and the presence of IgE antibody-bound polypeptides comprising the amino acid sequences defined above in (E4), (E9), (E11), (E19), (E31), (E36), (E37), (E43) and (E44) can be confirmed with laser beam. Examples of this method include a basophil activation test (BAT) which is a method in which a surface antigen CD203c that appears when basophils are activated by the contact of the polypeptides comprising the amino acid sequences defined above in (E4), (E9), (E11), (E19), (E31), (E36), (E37), (E43) and (E44) is detected. Another example includes a histamine release test (HRT) which examines whether histamine is released by further contacting the polypeptides comprising the amino acid sequences defined above in (E4), (E9), (E11), (E19), (E31), (E36), (E37), (E43) and (E44) with blood cells in a sample.

The polypeptides comprising the amino acid sequences defined above in (E4), (E9), (E11), (E19), (E31), (E36), (E37), (E43) and (E44) are antigens that specifically bind to IgE antibodies from allergic patients. Therefore, when binding between an IgE antibody from a subject and the antigen is detected, an indicator of the fact that the subject is allergic, also including cross-reactivity, is provided. In order to facilitate the synthesis of the polypeptides comprising the amino acid sequences defined above in (E4), (E9), (E11), (E19), (E31), (E36), (E37), (E43) and (E44) using, for example, E. coli, sequences may be added so as to flank the epitope to increase the sequence length. In such a case, when binding between an IgE antibody from a subject and the amino acid sequences defined above in (E4), (E9), (E11), (E19), (E31), (E36), (E37), (E43) and (E44) is detected, an indicator of the fact that the subject is allergic, also including cross-reactivity, is also provided. Therefore, any sequence may be added so as to flank the amino acid sequences defined above in (E4), (E9), (E11), (E19), (E31), (E36), (E37), (E43) and (E44) which are epitopes.

The present invention further provides a kit for diagnosing an allergy, comprising at least one of polypeptide comprising the amino acid sequences defined above in (E4), (E9), (E11), (E19), (E31), (E36), (E37), (E43) and (E44). The diagnosis kit of this invention may be used in the aforementioned method for providing an indicator for diagnosing an allergy or in a diagnosis method as described later. The diagnosis kit of this invention may comprise not only the at least one of polypeptide comprising the amino acid sequences defined above in (E4), (E9), (E11), (E19), (E31), (E36), (E37), (E43) and (E44), but also an anti-IgE antibody labeled with an enzyme and a chromogenic or luminescent substrate serving as a substrate for the enzyme. Also, a fluorescent-labeled anti-IgE antibody may be used. In the diagnosis kit of this invention, the polypeptide comprising the amino acid sequences defined above in (E4), (E9), (E11), (E19), (E31), (E36), (E37), (E43) and (E44) may be provided in a state immobilized on a carrier. The diagnosis kit of this invention may also be provided together with instructions on the procedure for diagnosis or a package containing said instructions.

In another embodiment, the aforementioned diagnosis kit comprises a companion diagnostic agent for an allergy. The companion diagnostic agent is used for identifying patients expected to respond to pharmaceutical products or identifying patients having the risk of severe adverse reactions to pharmaceutical products, or for studying the reactivity of pharmaceutical products in order to optimize treatment using the pharmaceutical products. Here, the optimization of treatment includes, for example, determination of dosage and administration, judgment regarding discontinuation of administration, and confirmation of an allergen component that is used to cause immunological tolerance.

The present invention further provides a composition for diagnosing an allergy, comprising at least one of polypeptide comprising the amino acid sequences defined above in (E4), (E9), (E11), (E19), (E31), (E36), (E37), (E43) and (E44). The diagnosis composition of this invention can be used in a diagnosis method as described below. The diagnosis composition of this invention may further comprise a pharmaceutically acceptable carrier and/or additives commonly used with the polypeptide of this invention depending on the need.

In one embodiment, the present invention provides a method for diagnosing an allergy in a subject, the method comprising:
(i) contacting a sample obtained from the subject with an antigen;
(ii) detecting binding between an IgE antibody present in the sample from the subject and the antigen; and
(iii) when the binding between the IgE antibody in the subject and the antigen is detected, diagnosing the subject as being allergic;
wherein the antigen is at least one of polypeptides defined as the polypeptides comprising the amino acid sequences defined above in (E4), (E9), (E11), (E19), (E31), (E36), (E37), (E43) and (E44). In this method, the steps (i) and (ii) are performed as described above regarding the corresponding steps of the method for providing an indicator for diagnosing an allergy.

In another embodiment, the present invention provides a method for diagnosing an allergy in a subject, the method comprising administering to the subject at least one of the polypeptides comprising the amino acid sequences defined above in (E4), (E9), (E11), (E19), (E31), (E36), (E37), (E43) and (E44). This method may be performed in the form of a skin test characterized by applying the polypeptide comprising the amino acid sequences defined above in (E4), (E9), (E11), (E19), (E31), (E36), (E37), (E43) and (E44) onto the skin. Examples of the skin test include various forms of tests, such as: a prick test in which a diagnosis composition is applied onto the skin and then a tiny prick to such an extent as not to provoke bleeding is made in the skin to allow the polypeptides comprising the amino acid sequences defined above in (E4), (E9), (E11), (E19), (E31), (E36), (E37), (E43) and (E44) to penetrate the skin, thereby observing a skin reaction; a scratch test in which a diagnosis composition is applied onto the skin and then the skin is lightly scratched to observe a reaction; a patch test in which a diagnosis composition in the form of cream, ointment, etc. is applied onto the skin to observe a reaction; and an intracutaneous test in which the polypeptides comprising the amino acid sequences defined above in (E4), (E9), (E11), (E19), (E31), (E36), (E37), (E43) and (E44) are administered intracutaneously to observe a reaction. If a skin reaction such as swelling occurs in a skin portion to which the polypeptide comprising the amino acid sequences defined above in (E4), (E9), (E11), (E19), (E31), (E36), (E37), (E43) and (E44) has been applied, the subject of interest is diagnosed as having an allergy. The amount of the aforementioned polypeptide to be applied to the skin in such tests can be, for example, not more than 100 µg per dose.

In the process of allergy diagnosis, an oral load test aiming to identify an antigen and to examine the degree of symptoms from antigen consumption is often adopted. At least one of the polypeptides comprising the amino acid sequences defined above in (E4), (E9), (E11), (E19), (E31), (E36), (E37), (E43) and (E44) can be used as an active ingredient for an oral load test to diagnose an allergy. Here, the polypeptide to be used in the oral load test may be a polypeptide that has been expressed and purified and may be a polypeptide that has been expressed in raw materials or processed products, such as rice-based vaccine expressing pollen allergens which are obtained by transforming rice with a gene of a cedar pollen antigen and expressing the polypeptide in the rice.

In one embodiment, the diagnosis composition or the diagnosis kit described above may be used for a prick test, a scratch test, a patch test, an intracutaneous test or the like.

In still another embodiment, the present invention provides at least one of the polypeptides comprising the amino acid sequences defined above in (E4), (E9), (E11), (E19), (E31), (E36), (E37), (E43) and (E44), intended for use in the diagnosis of an allergy.

In still another embodiment, the present invention provides use of at least one of the polypeptides comprising the amino acid sequences defined above in (E4), (E9), (E11), (E19), (E31), (E36), (E37), (E43) and (E44) in the production of a diagnostic agent for an allergy.

In this subsection, the allergy to be diagnosed can be allergies to the aforementioned polypeptides comprising the amino acid sequences defined above in (E4), (E9), (E11), (E19), (E31), (E36), (E37), (E43) and (E44). Thus, diagnosis of the allergy including detection of the allergy and provision of a diagnostic indicator can be diagnosis of not only an allergy to a single one of the polypeptides comprising the amino acid sequences defined above in (E4), (E9), (E11), (E19), (E31), (E36), (E37), (E43) and (E44), but also allergies including cross-reactivity.

### Composition and treatment method (2)

The present invention provides a composition comprising at least one of the polypeptides comprising the amino acid sequences defined above in (E4), (E9), (E11), (E19), (E31), (E36), (E37), (E43) and (E44).

In one embodiment, the composition of the present invention is a pharmaceutical composition. In one embodiment, the composition of the present invention is a quasi-pharmaceutical composition or a nonpharmaceutical composition (e.g., a cosmetic composition and a food composition).

In one embodiment, the aforementioned composition is used for the treatment of an allergy. The treatment of an allergy increases the limit amount of a polypeptide in which the polypeptide does not develop even if the antigen is incorporated into the body, and finally aims for the state where the allergy does not develop by the common amount of the polypeptide to be consumed (remission).

The present invention also provides a method for treating an allergy, the method comprising administering at least one of the polypeptides comprising the amino acid sequences defined above in (E4), (E9), (E11), (E19), (E31), (E36), (E37), (E43) and (E44) to a patient in need of a treatment for an allergy.

In another embodiment, the present invention provides at least one of the polypeptides comprising the amino acid sequences defined above in (E4), (E9), (E11), (E19), (E31), (E36), (E37), (E43) and (E44), intended for use in the treatment for an allergy. In yet another embodiment, the present invention provides use of at least one of the polypeptides comprising the amino acid sequences defined above in (E4), (E9), (E11), (E19), (E31), (E36), (E37), (E43) and (E44) for the production of a therapeutic agent for an allergy.

In the process of allergy treatment, a hyposensitization therapy aiming to induce immunological tolerance by administering an antigen to a patient is often adopted. The at least one of the polypeptides comprising the amino acid sequences defined above in (E4), (E9), (E11), (E19), (E31), (E36), (E37), (E43) and (E44) can be used as an active ingredient for hyposensitization therapy for an allergy. Here, the antigen to be used in the hyposensitization therapy may be a polypeptide that has been expressed and purified and may be a polypeptide that has been expressed in raw materials or processed products as rice, such as rice-based vaccine expressing pollen allergens.

The administration route, administration dose, frequency and/or period of the composition of this invention, and other ingredients to be contained in the composition, and the dosage form can be as described above in the subsection titled "Composition and treatment method (1)". In the case of using the polypeptides comprising the amino acid sequences defined above in (E4), (E9), (E11), (E19), (E31), (E36), (E37), (E43) and (E44), for example, the dose to an adult patient may be a dose of not more than 100 µg per dose.

In this subsection, the allergy to be treated can be allergies to the polypeptides comprising the amino acid sequences defined above in (E4), (E9), (E11), (E19), (E31), (E36), (E37), (E43) and (E44). Thus, treatment of the allergy can be treatment of not only an allergy to a single allergen, but also allergies including cross-reactivity.

### Tester Composition (2)

The present invention provides a tester composition comprising an antibody for at least one of the polypeptides comprising the amino acid sequences defined above in (E4), (E9), (E11), (E19), (E31), (E36), (E37), (E43) and (E44).

The antibody can be prepared by a conventional method. For example, the antibody may be prepared by immunizing a mammal such as rabbit with the polypeptides comprising the amino acid sequences defined above in (E4), (E9), (E11), (E19), (E31), (E36), (E37), (E43) and (E44). The antibody may be an Ig antibody, a polyclonal antibody, a monoclonal antibody, or an antigen-binding fragment thereof (e.g., Fab, F(ab')₂, Fab').

Further, in the aforementioned tester composition, the antibody may be provided in a form bound to a carrier. The type of the carrier is not particularly limited as long as it is usable for detection of binding between an antibody and the polypeptides comprising the amino acid sequences defined above in (E4), (E9), (E11), (E19), (E31), (E36), (E37), (E43) and (E44). Any given carrier known to those skilled in the art can be used. Also, the antibody for the polypeptides comprising the amino acid sequences defined above in (E4), (E9), (E11), (E19), (E31), (E36), (E37), (E43) and (E44) is preferably an antibody for polypeptides having the same amino acid sequences as the epitope and the important amino acid described above in the subsection titled "Epitope of antigen". This can attain a tester composition that can also detect cross-reactivity.

Examples of a method for determining the presence or absence of the polypeptides comprising the amino acid sequences defined above in (E4), (E9), (E11), (E19), (E31), (E36), (E37), (E43) and (E44) include the following methods:
a method in which a prepared tester composition comprising an antibody is contacted with a sample obtained from a raw material, a processed product, etc., ELISA or the like is used to detect whether there is a binding between the antibody and the polypeptides comprising the amino acid sequences as defined above in (E4), (E9), (E11), (E19), (E31), (E36), (E37), (E43) and (E44) in the sample, and if the binding between the antibody and the polypeptides comprising the amino acid sequences defined above in (E4), (E9), (E11), (E19), (E31), (E36), (E37), (E43) and (E44) is detected, it is determined that the polypeptides comprising such amino acid sequences are contained in the raw material, the processed product, etc. of interest (the "method for determining the presence or absence of a polypeptide" comprises confirming that the polypeptide is eliminated or reduced when the binding between the antibody and the polypeptides comprising the amino acid sequences defined above in (E4), (E9), (E11), (E19), (E31), (E36), (E37), (E43) and (E44) is reduced); and
a method in which filter paper is impregnated with a raw material, a processed product, etc. and reacted with an antibody solution so as to detect the polypeptides comprising the amino acid sequences defined above in (E4), (E9), (E11), (E19), (E31), (E36), (E37), (E43) and (E44) contained therein.

Another embodiment of the present invention includes a tester composition for determining the presence or absence of a polypeptide comprising the amino acid sequences defined above in (E4), (E9), (E11), (E19), (E31), (E36), (E37), (E43) and (E44) of an allergy in an object of interest, the tester composition comprising a primer appropriate for a polypeptide having an amino acid sequence where epitope and important amino acid are identical. The primer is not limited and may be designed so as to comprise, for example, a portion of the nucleotide sequence of a nucleic acid encoding any of the amino acid sequences defined above in (E4), (E9), (E11), (E19), (E31), (E36), (E37), (E43) and (E44), or a complementary strand thereof. Alternatively, the primer may be designed so as to be the nucleotide sequence of a region upstream of a portion encoding polypeptides having the same amino acid sequences as an epitope that is any of the amino acid sequences defined above in (E4), (E9), (E11), (E19), (E31), (E36), (E37), (E43) and (E44), and an important amino acid, in nucleic acids encoding proteins comprising the polypeptides having the same amino acid sequences as the epitope and the important amino acid, or the nucleotide sequence of a complementary strand of a region downstream of the portion encoding polypeptides having the same amino acid sequences as the epitope and the important amino acid. Examples of such a primer include a primer which is a portion of at least one nucleotide sequence selected from the group consisting of SEQ ID NOs: 1, 3, 5, 7, 9, 11, 13, 15, 17, 19, 21, 23, 25 and 27 and/or a primer which is a portion of a sequence complementary to at least one nucleotide sequence selected from the group consisting of SEQ ID NOs: 1, 3, 5, 7, 9, 11, 13, 15, 17, 19, 21, 23, 25 and 27. Here, the position of the epitope in the full-length sequence of an antigen is as defined in Table 2 on the basis of the results of Examples. Particularly, when mRNA is of interest, the tester composition has a complementary primer of a poly-A tail.

For example, DNA is amplified by PCR (Polymerase Chain Reaction) including RT-PCR using templated DNA or mRNA obtained from a sample and the aforementioned primer, and the presence or absence of a nucleic acid encoding the amino acid sequences defined above in (E4), (E9), (E11), (E19), (E31), (E36), (E37), (E43) and (E44) in the sequence of the amplified DNA is determined to determine the presence or absence of the antigen comprising the aforementioned (E4), (E9), (E11), (E19), (E31), (E36), (E37), (E43) and (E44). Amplification methods by PCR for mRNA of interest can be exemplified by RACE. When one of amino acid sequences encoded by three possible open reading frames in the amplified DNA comprises any of the amino acid sequences defined above in (E4), (E9), (E11), (E19), (E31), (E36), (E37), (E43) and (E44), it is determined that the antigen is present. When no DNA is amplified, it is determined that the antigen is absent.

In one embodiment, the aforementioned tester composition is used to determine the presence or absence of the polypeptides comprising the amino acid sequences defined above in (E4), (E9), (E11), (E19), (E31), (E36), (E37), (E43) and (E44) in raw materials or processed products of interest in a food production line. The raw material may be a cooking ingredient or may be a cosmetic raw material, a pharmaceutical raw material or the like. The processed product may be an edible processed product or may be a cosmetic, a pharmaceutical product or the like. The tester composition may also be used for search for organism species contained in raw materials, may be used for quality inspection of production lines and pre-shipment products by manufacturers, or may be used for self-checking of the presence or absence of an antigen in raw materials or processed products of interest by consumers or users.

### Method for determining presence or absence of polypeptide (2)

The present invention includes a method for determining the presence or absence of the polypeptides comprising the amino acid sequences defined above in (E4), (E9), (E11), (E19), (E31), (E36), (E37), (E43) and (E44) in a raw material or a processed product. This method comprises detecting a polypeptide having the whole or a portion of any of the amino acid sequences of the polypeptides comprising the amino acid sequences defined above in (E4), (E9), (E11), (E19), (E31), (E36), (E37), (E43) and (E44) in a raw material or a processed product.

In one embodiment, the method of the present invention comprises a step of determining the presence or absence of at least one of the polypeptides comprising the amino acid sequences defined above in (E4), (E9), (E11), (E19), (E31), (E36), (E37), (E43) and (E44) in a substance of interest, the step comprising contacting an antibody for the at least one of the polypeptides comprising the amino acid sequences defined above in (E4), (E9), (E11), (E19), (E31), (E36), (E37), (E43) and (E44) with a raw material or a processed product (including a liquid).

Such an antibody, definition of the raw material or the processed product, a method for preparing the antibody, a method for contacting the antibody with a raw material or a processed product, the binding between the antibody and the antigen, etc. are as described above in the subsection titled "Tester composition (2)".

Alternatively, the aforementioned method for determining the presence or absence of an antigen also includes a embodiment in which a portion of an epitope in the polypeptides comprising the amino acid sequences defined above in (E4), (E9), (E11), (E19), (E31), (E36), (E37), (E43) and (E44) contained in an antigen is detected. The "portion of an epitope" is preferably at least 4 amino acid residues, at least 6 amino acid residues or at least 8 amino acid residues. Detection of the portion of an epitope can be carried out by a known method for detecting a particular amino acid sequence that is a portion of a polypeptide. For example, a method is possible in which a protein in a raw material, a processed product, etc. (e.g., a cooking ingredient) of interest is cleaved with a digestive enzyme for an antigen elimination treatment and separated by HPLC or the like, and whether a peak of an any given epitope peptide is reduced by the antigen elimination treatment is measured. Alternatively, the presence or absence of an antigen comprising any of the polypeptides comprising the amino acid sequences defined above in (E4), (E9), (E11), (E19), (E31), (E36), (E37), (E43) and (E44) in a substance of interest may be determined using an antibody that recognizes a portion of the polypeptides comprising the amino acid sequences defined above in (E4), (E9), (E11), (E19), (E31), (E36), (E37), (E43) and (E44).

Antigen-free raw materials and the like (2)

The present invention provides a food raw material or an edible processed product in which at least one of the polypeptides comprising the amino acid sequences defined above in (E4), (E9), (E11), (E19), (E31), (E36), (E37), (E43) and (E44) is eliminated or reduced.

The method for eliminating or reducing the antigen of the present invention in raw materials or processed products is not limited. The elimination or reduction of the antigen may be conducted by any method, as long as the method permits the elimination or reduction of the polypeptides comprising the amino acid sequences defined above in (E4), (E9), (E11), (E19), (E31), (E36), (E37), (E43) and (E44). For example, the techniques described above in the subsection titled "Antigen-free food and the like" may be used.

Elimination or reduction of at least one of the polypeptides comprising the amino acid sequences defined above in (E4), (E9), (E11), (E19), (E31), (E36), (E37), (E43) and (E44) may be achieved by eliminating or reducing the whole amino acid sequence or may be achieved by cleaving or removing the the amino acid sequence moieties defined above in (E4), (E9), (E11), (E19), (E31), (E36), (E37), (E43) and (E44) from the antigen protein. The "removal" includes deletion and modification of the whole or a portion of the sequence moieties defined above in (E4), (E9), (E11), (E19), (E31), (E36), (E37), (E43) and (E44).

For example, the raw material in which the polypeptides comprising the amino acid sequences defined above in (E4), (E9), (E11), (E19), (E31), (E36), (E37), (E43) and (E44) are eliminated or reduced may be obtained by preparing a raw material in which the polypeptides comprising the amino acid sequences defined above in (E4), (E9), (E11), (E19), (E31), (E36), (E37), (E43) and (E44) are no longer expressed, using a genetic modification technique. Any technique known to those skilled in the art can be used as a genetic modification knock-out technique.

The processed product in which the polypeptides comprising the amino acid sequences defined above in (E4), (E9), (E11), (E19), (E31), (E36), (E37), (E43) and (E44) are eliminated or reduced may be a processed product of the raw material in which the polypeptides comprising the amino acid sequences defined above in (E4), (E9), (E11), (E19), (E31), (E36), (E37), (E43) and (E44) are eliminated or reduced, such as powdered milk obtained with a protein digest as a raw material. In the case of using an ordinary raw material, a treatment for removing or reducing the polypeptides comprising the amino acid sequences defined above in (E4), (E9), (E11), (E19), (E31), (E36), (E37), (E43) and (E44) is performed before, during or after preparation of a processed product. The "preparation of the processed product" means, for example, preparation of a processed food product (e.g., processed products of wheat, for example, bread, pasta, wheat needle and cake) from a food raw material (e.g., wheat).

The techniques described in the subsection titled "Antigen-free food and the like" may be used as methods for removing or reducing the polypeptides comprising the amino acid sequences defined above in (E4), (E9), (E11), (E19), (E31), (E36), (E37), (E43) and (E44) in processed products obtained with an ordinary raw materials. Examples of the method for cleaving the polypeptides comprising the amino acid sequences defined above in (E4), (E9), (E11), (E19), (E31), (E36), (E37), (E43) and (E44) include a method in which a polypeptide is treated by cleavage with a particular digestive enzyme.

### Method for producing raw material or processed product in which antigen is eliminated or reduced (2)

The present invention provides a method for producing a raw material or a processed product in which at least one of the polypeptides comprising the amino acid sequences defined in (E4), (E9), (E11), (E19), (E31), (E36), (E37), (E43) and (E44) is eliminated or reduced, the method comprising the step of confirming that the antigen is eliminated or reduced, in a production process of the processed product.

In the production method, elimination or reduction of the polypeptides comprising the amino acid sequences defined in (E4), (E9), (E11), (E19), (E31), (E36), (E37), (E43) and (E44) means that at least one of the polypeptides comprising the amino acid sequences defined above in (E4), (E9), (E11), (E19), (E31), (E36), (E37), (E43) and (E44) is eliminated or reduced, or the sequence moieties defined in (E4), (E9), (E11), (E19), (E31), (E36), (E37), (E43) and (E44) are cleaved or removed from the antigen.

A technique of confirming that the polypeptide is eliminated or reduced in the production process of the raw material or processed product is not particularly limited, and any technique capable of detecting at least one of the polypeptides comprising the amino acid sequences defined above in (E4), (E9), (E11), (E19), (E31), (E36), (E37), (E43) and (E44) may be used. For example, the presence or absence of the polypeptide in the raw material or processed product may be confirmed from the binding activity of an antibody for at least one of the polypeptides comprising the amino acid sequences defined above in (E4), (E9), (E11), (E19), (E31), (E36), (E37), (E43) and (E44) against a sample containing a material resulting from the production process of the raw material or processed product. Details of such a method are as described above in the subsection titled "Diagnosis kit and method". Thus, in the production method, the "IgE antibody from a subject" described above in the subsection titled "Diagnosis kit and method (2)" is replaced with the "antibody for at least one of the polypeptides comprising the amino acid sequences defined above in (E4), (E9), (E11), (E19), (E31), (E36), (E37), (E43) and (E44)", and the "antigen" "polypeptide" described above in the subsection titled "Diagnosis kit and method (2)" is replaced with the "sample containing a material resulting from the production process of the processed product". The techniques described above in the subsection titled "Diagnosis kit and method (2)" can be used to confirm that the antigen is eliminated or reduced in the production process of the processed product. The tester compositions described above in the subsection titled "Tester composition (2)" can also be used.

The present invention also relates to use of a kit in a method for diagnosing an allergy, use of a kit for diagnosing an allergy and/or for providing an indicator for diagnosis, a composition for use in a method for diagnosing an allergy, use of a composition for diagnosing an allergy and/or for providing an indicator for diagnosis, a method for diagnosing an allergy and/or for a providing an indicator for diagnosis, use of an antigen (protein antigen or epitope antigen) in a method for detecting the presence or absence of an IgE antibody in a sample obtained from a subject (an organism such as a human), an antigen (protein antigen or epitope antigen) for use in treatment of an allergy, a kit or a composition for detecting the binding between an IgE antibody in a sample obtained from a subject (an organism such as a human) and an antigen (protein antigen or epitope antigen), comprising the antigen (protein antigen or epitope antigen), and use of a kit or a composition for detecting the binding between an IgE antibody in a sample obtained from a subject (an organism such as a human) and an antigen (protein antigen or epitope antigen), the kit or the composition comprising the antigen (protein antigen or epitope antigen). Each term such as the "antigen" is as mentioned above.

The following describes examples of the present invention. The technical scope of this invention is not limited by these examples.

### Example 1: Confirmation of a protein pattern

Proteins contained in wheat (Triticum aestivum) were investigated using a two-dimensional electrophoresis method described below.

### Protein extraction

Extraction and purification of proteins contained in wheat were carried out as follows. The proteins were extracted by adding mammalian cell lysis kit; MCL1 (produced by Sigma-Aldrich Co. LLC) to wheat flour. The constituents of the mammalian cell lysis kit; MCL1 are as mentioned below.
50 mM Tris-HCl pH 7.5
1mM EDTA
250 mM NaCl
0.1% (w/v) SDS
0.5% (w/v) Deoxycholic acid sodium salt
1% (v/v) Igepal CA-630 (surfactant (Octylphenoxy)polyethoxyethanol produced by Sigma-Aldrich Co. LLC)

### Appropriate amount of Protease Inhibitor

Thereafter, the precipitation procedure was repeated twice using 2D-CleanUP Kit (produced by GE). In the first round of precipitation, the collected liquid protein extract was precipitated by adding TCA (trichloroacetic acid) thereto and the precipitated product produced by this procedure (TCA-precipitated product) was collected. In the second round of precipitation, the TCA-precipitated product collected above was further precipitated by adding acetone thereto and the precipitated product (sample) produced by this procedure was collected.

### Preparation of a sample solution

Part of the collected sample (50 µg on a protein weight basis) was dissolved in 150 µL of a DeStreak Rehydration Solution (produced by GE), which is a swelling buffer for first-dimensional isoelectric focusing gels, thereby obtaining a sample solution for first-dimensional isoelectric focusing (sample solution for swelling). The constituents of the DeStreak Rehydration Solution are as mentioned below.
7M thiourea
2M urea
4% (w/v) of CHAPS
0.5% (v/v) IPG buffer; produced by GE
Moderate amount of BPB (bromophenol blue)

### Penetration of the sample into first-dimensional isoelectric focusing gels

First-dimensional isoelectric focusing gel strips (Immobiline Drystrip IPG gels (pH3-10NL); produced by GE) were immersed in 140 µL of the foregoing sample solution for first-dimensional isoelectric focusing (sample solution for swelling) and impregnated with the solution at room temperature overnight.

In this example, an IPGphor electrophoresis system produced by GE was used.

An electrophoresis tray was filled with silicone oil. Filter paper moisten with water was positioned at both ends of the gel strips impregnated with the sample, and the gel strips were set in the electrophoresis tray such that the gel strips were covered with silicone oil. Electrodes were placed on the gel strips with the filter paper intervening therebetween.

The maximum current of the isoelectric focusing system was set to 75 µA per gel strip, and the first-dimensional isoelectric focusing was carried out according to the following voltage program: (1) a constant voltage step was performed at a constant voltage of 300 V until the volt-hours reached 750 Vhr (the current variation width during electrophoresis for 30 minutes before the end of this step was 5 µA); (2) the voltage was increased gradually to 1000 V for 300 Vhr; (3) the voltage was further increased gradually to 5000 V for 4500 Vhr; and then (4) the voltage was held at a constant voltage of 5000 V until the total Vhr reached 12000.

### SDS equilibration of isoelectric focusing gels

After the aforementioned first-dimensional isoelectric focusing was done, the gel strips were taken out of the isoelectric focusing system, immersed in an equilibration buffer containing a reducing agent, and shaken at room temperature for 15 minutes. The constituents of the equilibration buffer containing the reducing agent are as mentioned below.
100 mM Tris-HCl (pH 8.0)
6M urea
30% (v/v) glycerol
2% (w/v) SDS
1% (w/v) DTT

Next, the equilibration buffer containing the reducing agent was removed, and then the gel strips were immersed in an equilibration buffer containing an alkylating agent and shaken at room temperature for 15 minutes to obtain SDS-equilibrated gels. The constituents of the equilibration buffer containing the alkylating agent are as mentioned below.
100 mM Tris-HCl (pH 8.0)
6M urea
30% (v/v) glycerol
2% (w/v) SDS
2.5% (w/v) iodoacetamide

### Second-dimensional SDS-PAGE

In this example, the XCell SureLock Mini-Cell electrophoresis system produced by Life Technologies was used. The second-dimensional electrophoresis gels used were NuPAGE 4-12% Bis-Tris Gels produced by Life Technologies. Also, an electrophoresis buffer composed of the following constituents was prepared and used.
50 mM MOPS
50 mM Tris base
0.1% (w/v) SDS
1 mM EDTA

Further, an agarose solution for gel adhesion was used in this example, which was prepared by dissolving 0.5% (w/v) Agarose S (produced by Nippon Gene Co., Ltd.) and a moderate amount of BPB (bromophenol blue) in the electrophoresis buffer.

SDS-PAGE wells were washed well with the electrophoresis buffer, and then the buffer used for the washing was removed. Next, the washed wells were charged with the fully dissolved agarose solution for gel adhesion. Next, the SDS-equilibrated gel strips were immersed in agarose and closely adhered to second-dimensional electrophoresis gels using tweezers. After it was confirmed that agarose was fully fixed with the gels being closely adhered to each other, electrophoresis was performed at a constant voltage of 200 V for about 45 minutes.

### Fluorescent staining of gels

The gels were fluorescently stained with SYPRO Ruby (produced by Life Technologies).

First, an airtight container to be used was washed well in advance with 98% (v/v) ethanol. The electrophoresed second-dimensional electrophoresis gel strips were taken out of the SDS-PAGE system, placed onto the washed airtight container, and treated twice by immersion in 50% (v/v) methanol and 7% (v/v) aqueous solution containing acetic acid for 30 minutes. Then, a further immersion treatment was done for 10 minutes, with the solution being replaced by water. Next, the second-dimensional electrophoresis gel strips were immersed in 40 mL of SYPRO Ruby and shaken at room temperature overnight. Thereafter, the SYPRO Ruby was removed, and then the second-dimensional electrophoresis gel strips were washed with water and shaken in 10% (v/v) methanol and 7% (v/v) aqueous solution containing acetic acid for 30 minutes. Further shaking was done for at least 30 minutes, with the solution being replaced by water.

### Analysis

The second-dimensional electrophoresis gels obtained through the foregoing series of treatments were subjected to fluorescent image scanning on Typhoon 9500 (produced by GE). The results of the two-dimensional electrophoresis as to the proteins contained in the wheat are shown in Fig. 1. Molecular weight marker bands are found at the left of the photograph of the gel of each panel. The positions of the bands denote particular molecular weights (in KDa).

### Example 2: Identification of antigens by immunoblotting

Identification of antigens by immunoblotting was carried out by taking all the steps up to the step of "Second-dimensional SDS-PAGE" as described above in Example 1, followed by the steps of "Transfer to membrane", "Immunoblotting" and "Analysis" as described below.

### Transfer to membrane

Transfer to membrane was done using the following transfer system and transfer buffer.
Transfer system: XCell SureLock Mini-Cell and XCell II Blot Module (produced by Life Technologies)
Transfer buffer: NuPAGE Transfer Buffer (X20) (produced by Life Technologies), used in a form diluted 200-fold with milliQ water.

To be specific, proteins in the two-dimensional electrophoresis gels were transferred to a membrane (PVDF membrane) according to the following procedure.
(1) The PVDF membrane was immersed in 100% methanol followed by milliQ water, and then moved into the transfer buffer to hydrophilize the PVDF membrane.
(2) After sponge, filter paper, the gels treated by second-dimensional SDS-PAGE, the hydrophilized PVDF membrane, filter paper, and sponge were put in place in this order, the transfer system was energized at a constant voltage of 30 V for one hour.

### Immunoblotting

Immunoblotting of the membrane was carried out using, as a primary antibody, a serum sample from a patient with a wheat allergy (WDEIA cases and cases with general immediate type allergy, which requires no exercise for its development) or a serum sample from a non-wheat-allergic subject.

Immunoblotting of the membrane was carried out according to the following procedure.
(1) The transferred membrane was shaken in a 5% skim milk/PBST solution (a PBS buffer containing 0.1% Tween 20 nonionic surfactant) at room temperature for one hour.
(2) The membrane was left to stand in a solution of 4% primary antibody serum in 3% skim milk/PBST at room temperature for one hour.
(3) The membrane was washed with a PBST solution (5 min. × 3 times).
(4) The membrane was left to stand in a 1:1000 dilution of the secondary antibody, anti-human IgE-HRP (horseradish peroxidase), with a 3% skim milk/PBST solution at room temperature for one hour.
(5) The membrane was washed with a PBST solution (5 min. × 3 times).
(6) The membrane was left to stand in Pierce Western Blotting Substrate Plus (produced by Thermo Fisher Scientific) for 5 minutes.

### Analysis

The membrane obtained through the foregoing series of treatments was subjected to fluorescent image scanning on Typhoon 9500 (produced by GE).

The immunoblots obtained with the serum from the wheat-allergic patient were compared with those obtained with the control serum from the non-wheat-allergic subject. For protein present in wheat, in immunoblotting using the serum from the wheat-allergic patient, 16 spots were detected (Fig. 2), which are different from the spots detected when the serum of the non-wheat-allergic subject was used and different from those of the known wheat allergen proteins. As a result of analyzing the amino acid sequences in Example 3, spots 11 and 12 were found to be derived from the same protein. The isoelectric point of each spot is described in Table 1.

### Example 3: Mass spectrometry and identification of antigens

The amino acid sequences of the antigens that form the protein spots were identified by mass spectroscopy.

To be specific, protein extraction and mass spectroscopy were done by the following procedure.
(1) Wheat was subjected to protein extraction, two-dimensional electrophoresis and transfer to membrane by following the procedures described in Example 1 and 2, and the resulting membrane was stained by shaking in a solution of 0.008% Direct blue in 40% ethanol and 10% acetic acid.
(2) Then, the membrane was decolorized by repeating a 5-minute treatment with 40% ethanol and 10% acetic acid three times, washed with water for 5 minutes, and then dried by air.
(3) A protein spot of interest was cut out with a clean cutter blade and put into a centrifugal tube. The cut membrane was subjected to hydrophilization with 50 µL of methanol, followed by washing with 100 µL of water twice and then centrifugal cleaning. Thereafter, 20 µL of 20 mM NH₄HCO₃ and 50% acetonitrile were added.
(4) 1 µL of 1 µmol/µL lysyl endopeptidase (produced by WAKO) was added, and the solution was left to stand at 37°C for 60 minutes and then collected in a new centrifugal tube. After 20 µL of 20 mM NH₄HCO₃ and 70% acetonitrile were added to the membrane, the membrane was immersed therein at room temperature for 10 minutes, and the resulting solution was further collected. The solution was dissolved with 0.1% formic acid and 10 µL of 4% acetonitrile and transferred to a tube.
(5) The collected solution was dried under reduced pressure, dissolved with 15 µL of solution A (a 0.1% formic acid/4% acetonitrile solution), and analyzed by mass spectroscopy (ESI-TOF6600, produced by AB Sciex).
(6) Identification of proteins based on the MS data obtained with the mass spectrometer was done by searching the NCBI database.

### Results

The amino acid sequence of each spot was detected. Further, the mass spectroscopic data of each spot obtained on a mass spectrometer was analyzed in Uniprot. As a result, each spot was found to be the protein shown in Table 1.

Spots 11 and 12 or spots 15 and 16 are derived from the same protein. The immunoblot of Example 2 recognized these spots as separate spots because even the same amino acid sequence varied in isoelectric point and/or molecular weight due to a posttranslational modification such as sugar chain modification or modification at a phosphate group. The binding to IgE antibodies was confirmed in spite of such difference in posttranslational modification, indicating that the posttranslational modification has no influence.

### Example 4: Identification of epitopes

### Epitopes of allergen components of wheat

Identification of epitopes was carried out by the following procedure as to epitopes of allergen components of wheat.

### (A) Wheat epitope mapping (1)

Epitope mapping was carried out using a library of overlapping peptides (length: 15 amino acids) corresponding to the amino acid sequences identified as allergy components of wheat. Specifically, the library of overlapping peptides was prepared on the basis of the amino acid sequences of SEQ ID NOs: 2, 4, 6, 8, 10, 12, 14, 16, 18, 20, 22, 24, 26 and 28.

The peptides to be synthesized were shifted by 10 amino acids. Thus, each peptide had an overlap of 5 amino acids with each of the peptides previous and subsequent thereto.

For preparation of peptide arrays, the Intavis CelluSpots(TM) technique was used. Specifically, the peptide arrays were prepared by the following procedure: (1) synthesizing peptides of interest on amino-modified cellulose disks using an automated chemical synthesis apparatus (Intavis MultiPep RS), (2) dissolving the amino-modified cellulose disks to obtain a cellulose-bound peptide solution, and (3) spotting the cellulose-bound peptides onto coated glass slides. The details of each procedure are as described below.

### (1) Synthesis of peptide

Peptide synthesis was carried out in incremental steps through 9-fluorenylmethoxycarbonyl (Fmoc) chemical reaction on amino-modified cellulose disks in 384-well synthesis plates. Specifically, amino acids in which a Fmoc group is bonded to an amino group were activated in a solution of N,N'-diisopropylcarbodiimide (DIC) and 1-hydroxybenzotriazole (HOBt) in dimethylformamide (DMF) and added dropwise to the cellulose disks so that the Fmoc group-bound amino acids were bound to the amino groups on the cellulose disks (coupling). Unreacted amino groups were capped with acetic anhydride and washed with DMF. Furthermore, the Fmoc groups were eliminated from the amino groups of the amino acids bound to the amino groups on the cellulose disks by treatment with piperidine and washing with DMF. The amino acids bound to the amino groups on the cellulose disks were repetitively subjected to the coupling, the capping, and the Fmoc group elimination described above to elongate the amino terminus for peptide synthesis.

### (2) Dissolution of amino-modified cellulose disk

The peptides-bound cellulose disks of interest obtained above in the subsection titled "(1) Synthesis of peptide" were transferred to 96-well plates and treated with a side chain deprotection mixed solution of trifluoroacetic acid (TFA), dichloromethane, triisopropylsilane (TIPS), and water for deprotection of amino acid side chains. Then, the deprotected cellulose-bound peptides were dissolved in a mixed solution of TFA, perfluoromethanesulfonic acid (TFMSA), TIPS, and water and precipitated in tetrabutyl methyl ether (TBME), and the precipitate was resuspended in dimethyl sulfoxide (DMSO) and mixed with a mixed solution of NaCl, sodium citrate, and water to obtain a peptide solution for slide spotting.

### (3) Spotting of cellulose-bound peptide solution

The peptide solution for slide spotting obtained above in the subsection titled "(2) Dissolution of amino-modified cellulose disk" was spotted onto Intavis CelluSpots(TM) slides using Intavis Slide Spotting Robot, and the slides were dried to prepare peptide arrays.

The presence or absence of binding, to each peptide fragment, of an IgE antibody present in the serum of a wheat-allergic patient was measured through antigen-antibody reaction using the peptide arrays. The measurement was carried out according to the following procedure.
(1) The peptides were shaken at room temperature for 1 hour in Pierce Protein-Free (PBS) Blocking Buffer (produced by Thermo Fisher Scientific).
(2) The peptide arrays were shaken at overnight at 4°C in 2% serum/Pierce Protein-Free (PBS) Blocking Buffer (produced by Thermo Fisher Scientific).
(3) The peptide arrays were washed with PBST (a PBS buffer containing 3% Tween 20 nonionic surfactant) for 5 minutes (× 3).
(4) Anti-human IgE antibody-HRP (1:20,000, Pierce Protein-Free (PBS) Blocking Buffer (produced by Thermo Fisher Scientific)) was added thereto, and the peptide arrays were shaken at room temperature for 1 hour.
(5) The peptide arrays were washed with PBST for 5 minutes (× 3).
(6) Pierce ECL Plus Western Blotting Substrate (produced by Thermo Fisher Scientific) was added thereto, and the peptide arrays were shaken at room temperature for 5 minutes.
(7) The chemiluminescence of the peptides treated as described above in (1) to (6) was measured using Amersham Imager 600.

Chemiluminescence intensity in images obtained by the measurement described above in (7) was converted into a numeric value using ImageQuant TL (GE Healthcare). The second highest value among numeric values determined from images obtained from results about the serum of 5 non-wheat-allergic subjects was defined as the N2nd value. The N2nd value of each peptide was subtracted from numeric values determined from images obtained from results about the serum of 49 patients (32 WDEIA cases, 8 children cases with general immediate type allergy to wheat, and 9 adult cases). It was determined that a peptide having a value of at least 35,000 as the difference was a peptide bound to an IgE antibody in a patient-specific manner.

As a result, patient-specific IgE antibodies were confirmed to bind to the peptides (SEQ ID NOs: 281, 640, 776, 1257, 1895, 2154, 2217, 2545 and 2613) derived from spots 1-16 (spots 11 and 12 or spots 15 and 16 are derived from the same protein) excluding known epitopes.

### (B) Wheat epitope mapping (2): overlapping

On the basis of the sequences (SEQ ID NOs: SEQ ID NOS: 281, 640, 776, 1257, 1895, 2154, 2217, 2545 and 2613) of the peptides bound to an IgE antibody in serum in a patient-specific manner as described above in (A), a library of overlapping peptide fragments (length: 10 amino acids) was prepared using the sequences of the peptides and sequences in which sequences were added so as to flank each peptide in the amino acid sequences of allergy components comprising the sequences of the peptide. Epitope mapping was carried out using the library.

The peptides to be synthesized were shifted by one amino acid. Thus, each peptide had an overlap of 9 amino acids with each of the peptides previous and subsequent thereto.

The library was prepared by the same procedure as described above in (A), and the presence or absence of binding of an IgE antibody present in the serum of a patient to each peptide fragment was measured by the same technique as described above. Numeric values determined from images obtained as a result of carrying out only the procedures (1) and (4) to (6) described above in the subsection titled (3) Spotting of cellulose-bound peptide solution were used as control values. The control value of each peptide was subtracted from numeric values determined from images obtained from results about the serum of patients, and the obtained value of the difference was compared with values of the peptides serving as the basis of overlapping. It was determined that a peptide that lost or markedly decreased binding activity against IgE antibodies from patients as compared with the peptides shifted by one amino acid was a peptide having no binding activity against IgE antibodies.

Chemiluminescence intensity in images obtained by measurement was converted into a numeric value in the same manner as in (A). Numeric values determined from images obtained as a result of carrying out only the procedures (1) and (4) to (6) described above in the subsection titled (3) Spotting of cellulose-bound peptide solution (secondary antibody measurement values) were used as control values. The control value of each peptide was subtracted from numeric values determined from images obtained from results about the serum of patients. When values obtained using the sequences (SEQ ID NOs: 281, 640, 776, 1257, 1895, 2154, 2217, 2545 and 2613) serving as the basis of overlapping were defined as 100%, it was determined that: a peptide having a value of the difference that was less than 30% had no binding activity against IgE antibodies; a peptide having a value of the difference that was 30% or more and less than 50% had binding activity, albeit poor, against IgE antibodies; a peptide having a value of the difference that was 50% or more and less than 70% had binding activity, albeit somewhat poor, against IgE antibodies; and a peptide having a value of the difference that was 70% or more had equivalent or good binding activity against IgE antibodies and was thus a peptide had remaining binding activity against IgE antibodies.

This analysis found regions important for binding to IgE antibodies from patients, in the sequences serving as the basis of overlapping.

### (C) Wheat epitope mapping (3): alanine/glycine scanning

From the amino acid sequences identified above in (A), a library of peptide fragments in which amino-terminal amino acids were substituted one by one by alanine (or glycine when the original amino acid was alanine) according to a technique called alanine/glycine scanning (Non Patent Literature 5) was prepared by the same technique as described above. The presence or absence of binding of an IgE antibody present in the serum of a patient to each peptide fragment was measured by the same technique as described above. Amino acids at positions where the binding activity against IgE antibodies from patients was lost or markedly decreased by the substitution by alanine/glycine were confirmed as amino acids important for exertion of original antigenicity, or amino acids influencing exertion of original antigenicity. Amino acids at positions where the binding activity against IgE antibodies from patients was neither lost nor markedly decreased were confirmed as substitutable amino acids that were not important for exertion of original antigenicity.

Chemiluminescence intensity in images obtained by measurement was converted into a numeric value in the same manner as in (A). Numeric values determined from images obtained from secondary antibody measurement values were used as control values. The control value of each peptide was subtracted from numeric values determined from images obtained from results about the serum of 49 patients. When values obtained using the sequences (SEQ ID NOs: 281, 640, 776, 1257, 1895, 2154, 2217, 2545 and 2613) serving as the basis of alanine/glycine scanning were defined as 100%, it was determined that: a peptide having a value of the difference that was less than 30% had no binding activity against IgE antibodies; a peptide having a value of the difference that was 30% or more and less than 50% had binding activity, albeit poor, against IgE antibodies; a peptide having a value of the difference that was 50% or more and less than 70% had binding activity, albeit somewhat poor, against IgE antibodies; and a peptide having a value of the difference that was 70% or more had equivalent or good binding activity against IgE antibodies and was thus a peptide had remaining binding activity against IgE antibodies.

By analysis of the results of (A) to (C), common sequences important for exertion of original antigenicity were found in regions important for binding to IgE antibodies from patients, in the sequences serving as the basis of alanine/glycine scanning. The sequences of all 50 types of epitopes were identified, and the results were summarized in Table 2.

### Example 5: Confirmation of epitope cross-reactivity

Amino acids other than amino acids important for maintaining binding to IgE antibodies, in each of the epitope sequences found in the wheat proteins in Table 2 were defined as any given amino acid (X). NCBI was searched for proteins having the key sequences having the amino acid residues X in cooking ingredient allergens carried in common by each of the patients. As a result, an amino acid sequence contained in a sequence in each food described in the column "Food with which cross-reactivity was confirmed" in Table 2 was identified as one example.

The binding activity of peptides comprising the amino acid sequences described in the column "Synthesis sequence" and the rightmost column "SEQ ID NO" of Table 2 against IgE antibodies from patients having allergies to each food described in the column "Food with which cross-reactivity was confirmed" in Table 2 was confirmed by ELISA. The peptides were synthesized such that the peptides were N-terminally biotinylated by the Fmoc method.

To be specific, ELISA was carried out according to the following procedure.
(1) The concentrations of the biotinylated peptides were adjusted to 10 µg/mL with PBST (0.1% Tween 20).
(2) Each peptide solution was added at 20 µL to each well of a 384-well plate coated with streptavidin, and shaken at room temperature for one hour. After collection of the solution, the wells were washed with PBST five times.
(3) Pierce Protein-Free (PBS) Blocking Buffer (produced by Thermo) was added at 40 µL thereto and shaken at room temperature for one hour. After removal of the solution, the wells were washed with PBST five times.
(4) 2% serum/Canget Signal Solution I (produced by TOYOBO) was added at 20 µL thereto and shaken at room temperature for one hour. After removal of the solution, the wells were washed with PBST five times.
(5) A diluted secondary antibody solution (1:10000, Canget SignalSolution II (produced by TOYOBO)) was added at 20 µL thereto and shaken at room temperature for one hour. After removal of the solution, the wells were washed with PBST five times.
(6) 1-Step Ultra TMB-ELISA (produced by Thermo) was added at 20 µL thereto and shaken at room temperature for 15 minutes.
(7) 2 M H₂SO₄ was added at 20 µL thereto. Absorbance at 450 nm was measured.

Peptides having these amino acid sequences were prepared by the same procedure as described in Example 4(A), and the presence or absence of binding thereto of IgE antibodies present in the serum of an allergic patient and the serum of a nonallergic subject was measured. The serum of two nonallergic subjects was measured, and values obtained by dividing the measurement values by an average value thereof were used.

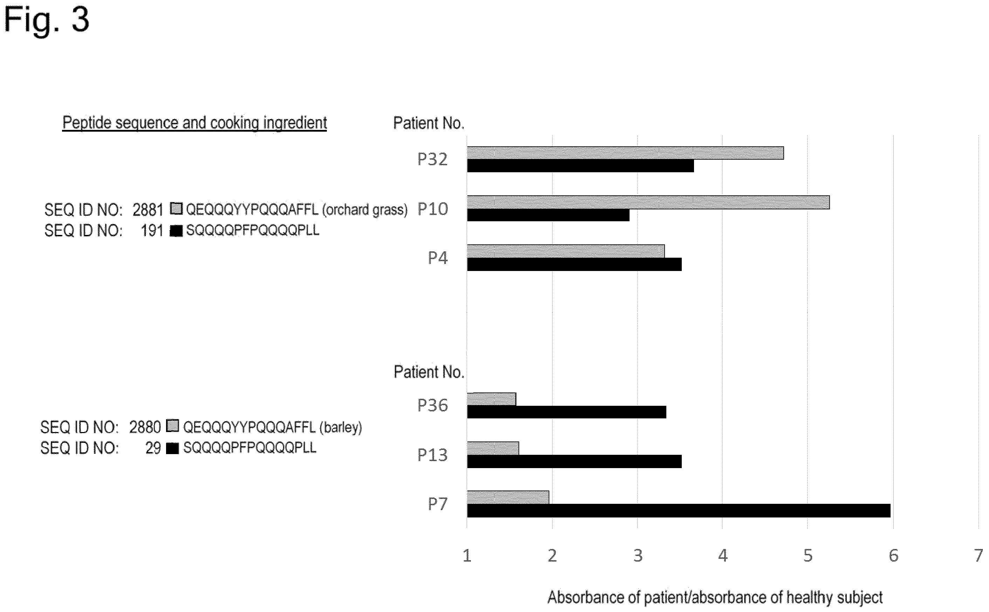

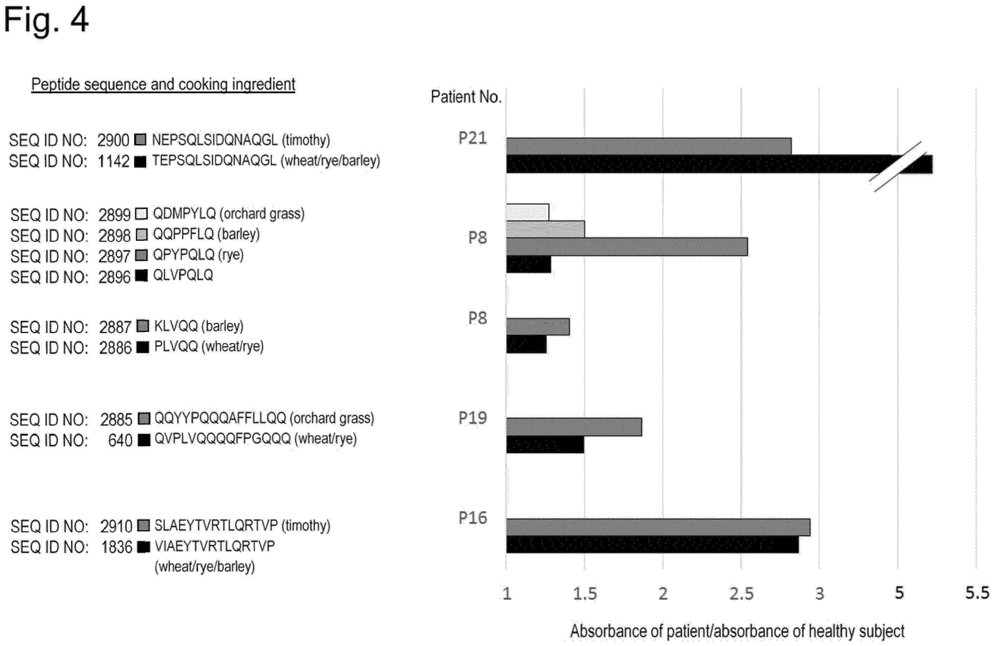

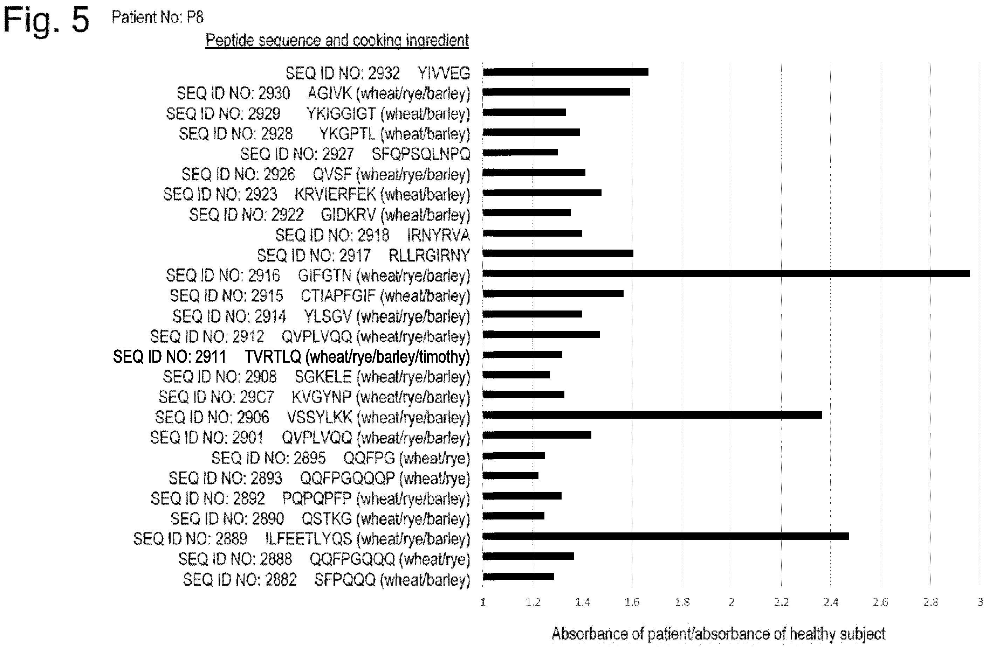

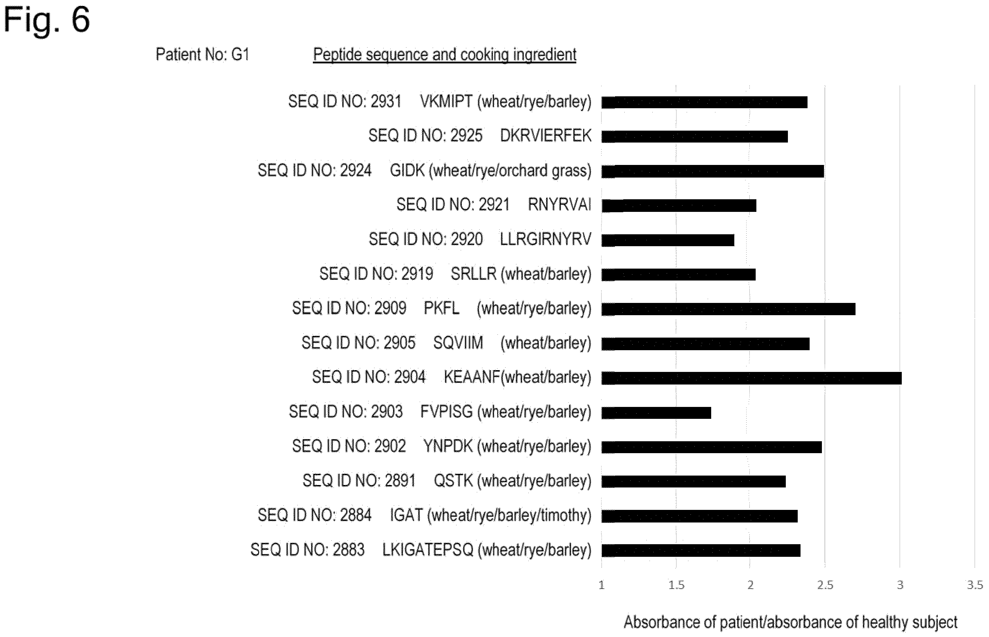

The results are shown in Figs. 3 to 6. The bar graph of each figure depicts "absorbance of the allergic patient / absorbance of the nonallergic patient (healthy subject)". The names of the cooking ingredients described in each graph are the names of cooking ingredients containing a polypeptide comprising an amino acid sequence serving as the basis of each synthetic sequence used.

As is evident from Figs. 3 to 6, all the polypeptides having the amino acid sequences described in the figures exhibited higher binding activity (larger than "l") against an IgE antibody from each allergic patient than against an IgE antibody in the serum of a nonallergic subject. Thus, these polypeptides were confirmed to have cross-reactivity. This indicates that their epitopes can be used for detecting cross-reactivity with antigens other than wheat antigens. This further supports that the "X" moiety can assume any given amino acid residue.

## Claims

1. A kit for diagnosing an allergy, comprising at least one of the following polypeptides (E4), (E9), (E11), (E19), (E31), (E36), (E37), (E43) and (E44) :
(E4) a polypeptide comprising at least one amino acid sequence selected from the amino acid sequences of SEQ ID NOs: 281-341 and 343-345;
(E9) a polypeptide comprising at least one amino acid sequence selected from the amino acid sequences of SEQ ID NOs: 640-705 and 2885-2888;
(E11) a polypeptide comprising at least one amino acid sequence selected from the amino acid sequences of SEQ ID NOs: 776-898 and 2892;
(E19) a polypeptide comprising at least one amino acid sequence selected from the amino acid sequences of SEQ ID NOs: 1257-1296;
(E31) a polypeptide comprising at least one amino acid sequence selected from the amino acid sequences of SEQ ID NOs: 1895-1976, 2912 and 2913;
(E36) a polypeptide comprising at least one amino acid sequence selected from the amino acid sequences of SEQ ID NOs: 2154-2216;
(E37) a polypeptide comprising at least one amino acid sequence selected from the amino acid sequences of SEQ ID NOs: 2217-2264 and 2917-2921;
(E43) a polypeptide comprising at least one amino acid sequence selected from the amino acid sequences of SEQ ID NOs: 2545-2612; and
(E44) a polypeptide comprising at least one amino acid sequence selected from the amino acid sequences of SEQ ID NOs: 2613-2630;

2. A composition for diagnosing an allergy, the composition comprising at least one of polypeptides (E4), (E9), (E11), (E19), (E31), (E36), (E37), (E43) and (E44) according to claim 1.

3. A method for providing an indicator for diagnosing an allergy in a subject, the method comprising the steps of:
(i) contacting a sample obtained from the subject with an antigen, wherein the sample is a solution comprising an IgE antibody;
(ii) detecting binding between the IgE antibody present in the sample obtained from the subject and the antigen; and
(iii) when the binding between the IgE antibody in the subject and the antigen is detected, an indicator of the fact that the subject is allergic is provided;
wherein the antigen is at least one of polypeptides (E4), (E9), (E11), (E19), (E31), (E36), (E37), (E43) and (E44) according to claim 1.

4. An antigen which is at least one of polypeptides (E4), (E9), (E11), (E19), (E31), (E36), (E37), (E43) and (E44) according to claim 1 and is causative of an allergy.

5. A composition comprising at least one antigen according to claim 4.

6. The composition according to claim 5, wherein the composition is intended for the treatment of an allergy.

7. A tester composition for determining the presence or absence of an antigen in a subject, the tester composition comprising an antibody that binds to at least one of polypeptides (E4), (E9), (E11), (E19), (E31), (E36), (E37), (E43) and (E44) according to claim 1.

8. A tester composition for determining the presence or absence of an antigen in an object of interest, the tester composition comprising at least one primer comprising a portion of the nucleotide sequence of a nucleic acid encoding any of polypeptides (E4), (E9), (E11), (E19), (E31), (E36), (E37), (E43) and (E44) according to claim 1, and/or a portion of a complementary strand thereof.

9. A tester composition for determining the presence or absence of an IgE antibody in a subject, the tester composition comprising polypeptides (E4), (E9), (E11), (E19), (E31), (E36), (E37), (E43) and (E44) according to claim 1.

10. A method for determining the presence or absence of polypeptides (E4), (E9), (E11), (E19), (E31), (E36), (E37), (E43) and (E44) according to claim 1 in a raw material or a processed product, comprising detecting the polypeptides (E4), (E9), (E11), (E19), (E31), (E36), (E37), (E43) and (E44) according to claim 1 in the raw material or the processed product.

11. A raw material or a processed product in which an antigen is eliminated or reduced, wherein the antigen is at least one of polypeptides (E4), (E9), (E11), (E19), (E31), (E36), (E37), (E43) and (E44) according to claim 1.

12. A method for producing a raw material or a processed product in which an antigen is eliminated or reduced, the method comprising the step of confirming that the antigen is eliminated or reduced, in a production process of the processed product, wherein the antigen is at least one of polypeptides (E4), (E9), (E11), (E19), (E31), (E36), (E37), (E43) and (E44) according to claim 1.
